# EUROPEAN PATENT APPLICATION

(11) **EP 3 981 394 A1**
(43) Date of publication of application: **13.04.2022**
(21) Application number: 20200566.6
(22) Date of filing: 07.10.2020
(51) Int. Cl.: A61K 31/00, A61K 31/19, A61K 31/192, A61K 31/194, A61K 31/41, A61K 31/415, A61K 31/4155, A61K 31/44, A61K 45/06, A61P 25/00, A61K 39/00

(54) **A SORTILIN ANTAGONIST FOR USE IN THE PREVENTION OR TREATMENT OF HEARING LOSS.**

(71) Applicant: Insusense ApS, 2100 Copenhagen (DK)
(72) Inventor: Kjølby, Mads Fuglsang, DK-2100 Copenhagen (DK); Nykjær, Anders, DK-2100 Copenhagen (DK); Little, Paul, DK-2100 Copenhagen (DK); Cases, Manuel, Reading, Berkshire RG4 7PD (GB)
(74) Representative: Gill Jennings & Every LLP

(57) **Abstract**

The present invention relates to the use of sortilin antagonists, in particular compounds of Formula (I), (II), (III), and (IV), in the treatment or prevention of hearing loss. Also provided are pharmaceutical compositions comprising the sortilin antagonists herein disclosed and methods for the treatment or prevention of hearing loss in a subject in need thereof.

## Description

### FIELD OF THE INVENTION

The present invention relates to the use of sortilin modulators, in particular compounds of Formula (I), (II), (III) and (IV), in the treatment or prevention of hearing loss.

### BACKGROUND

Hearing loss refers to either the partial, or total, acquired inability of an individual to hear. Accordingly, hearing loss can result in a severely reduced quality of life. Hearing loss may have a variety of causes, for example, the natural aging process, repeated exposure to loud noises or as a consequence of exposure to various toxins or pharmaceutical substances. Hearing loss is most commonly associated with damage to the hair cells, structures in the inner ear, which once damaged are unable to divide or regenerate themselves. Currently, there are no approved drugs for treating hearing loss. Current, symptomatic, treatment options for individuals suffering from hearing loss primarily revolve around the use of hearing aids or cochlear implants. Whilst these devices are able to improve the hearing of some individuals, not all individuals experience the desired effect. Additionally, such devices are deemed to be aesthetically displeasing and only treat the symptoms of hearing loss, and not the underlying disease pathology. Further, the available treatments are not useful for preventing or restoring the initial hearing loss nor preventing progression of hearing loss.

Sortilin (encoded by *SORT1*) is a type 1 membrane receptor in the vacuolar protein sorting 10 protein (VPS10P) family of sorting receptors, and is abundantly expressed in the central nervous system, the inner ear, and in some peripheral tissues involved in metabolic control^{1,2,3,4}. Sortilin has an amino acid sequence according to SEQ ID NO: 1 and comprises a signal peptide, a propeptide, the Vps10p domain, a 10cc domain (10CCa + 10CCb), a transmembrane domain and a large cytoplasmic tail. The luminal domain of sortilin has 6 potential *N*-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (proNGF), brain derived neurotrophic factor (proBDNF), and neurotrophin-3 (proNT3), respectively. In complex with the protein p75NTR (p75 neurotrophin receptor), sortilin has been reported to form the receptor for pro-neurotrophin-mediated apoptotic effects leading to degeneration and cell death in cellular and animal models^{5,6,7}.

There is an unmet need for new substances useful in the treatment, e.g. by means of delaying progression of symptoms, and prevention of hearing loss, which address the underlying causes of the disease and not just the symptoms.

### DESCRIPTION OF THE FIGURES

Figure 1 shows the mean maximal perilymph concentration of the compound exemplified in Example 8 in male Hartley guinea-pigs following trans-tympanic administration at either 0.1 mg/mL or 1 mg/mL at 1 h, 8 h and 72 h post dosing. Data is shown as Mean ± SD.
Figure 2 shows the mean maximal perilymph concentration of the compound exemplified in Example 8 in male Harley guinea-pigs following oral administration at 1 mg/mL, at 8 h and 24 h following three days of daily dosing. Data is shown as Mean ± SD.

### DISCLOSURE OF THE INVENTION

In a first aspect, the present invention provides a sortilin antagonist for use in the prevention or treatment of hearing loss.

Without wishing to be bound by theory, it is thought that the protein sortilin is upregulated in the ear of hearing loss patients. Sortilin can form a trimeric complex with the low-affinity nerve growth factor receptor (p75NTR) and a pro-neurotrophin, such as proNGF, proBDNF or proNT3, resulting in cell death of inner ear cells while at the same time preventing the conversion of the proform into its mature and trophic form¹. The present invention provides substances which interfere with the binding of sortilin to pro-neurotrophins, which: 1) prevents the trimeric apoptotic complex of sortilin, p75NTR and the pro-neurotrophin from forming, and 2) prevents sortilin mediated clearance of the pro-neurotrophins, providing a pool of substrate to be converted into the mature and trophic neurotrophin. Thus, sortilin-proneurotrophin interference can be used in protecting the ear from damage resulting from the aforementioned cell death.

It has surprisingly been found that sortilin antagonists, in particular compounds of Formula (I), (Ia), (II), (III), and (IV) may be useful in the treatment or prevention of hearing loss.

According to another aspect, the invention provides the use of a sortilin antagonist in the manufacture of a medicament for the prevention or treatment of hearing loss.

According to another aspect of the invention, there is provided a method of preventing or treating hearing loss by administering a therapeutically effective amount of a sortilin antagonist to a subject in need thereof.

The invention also provides a pharmaceutical formulation comprising the sortilin antagonist of the invention.

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" (used interchangeably herein) as used herein may also include any protein that is functionally related to sortilin, i.e. a sortilin-related molecule, for example, SorSC1, SorCS2, SorCS3 or SorLA/SORL1. Therefore, for the avoidance of doubt, any reference to the term "sortilin" in a use, method or composition of the invention, may not only refer to sortilin as defined above but may also be taken to optionally alternatively refer to a functionally related protein, such as SorCS1, SorCS2, SorCS3 or SorLA/SORL1. It is understood that the sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells.

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include proNGF, proBDNF, proNT3, proNT4, preferably the pro-neurotrophin is proNGF, proBDNF or proNT3, even more preferably the pro-neurotrophin is proNGF.

As used herein, the term "sortilin antagonist" refers to a substance (such as an antibody, protein, or other molecule) that interferes with, blocks, or otherwise attenuates the effect of, a sortilin protein binding to a pro-neurotrophin (e.g proNGF, proNT3, proBDNF) and preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not proNGF) and p75NTR can simultaneously bind the NGF domain of proNGF. However, the resulting trimeric complex may be of lower affinity for its receptor and as a result have significantly reduced capacity to stimulate apoptosis via the mechanism describe above. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency.

The sortilin antagonist herein disclosed is intended for the use in the prevention or treatment of hearing loss. The hearing loss may for example be noise-induced hearing loss, ototoxicity-induced hearing loss, age-induced hearing loss, idiopathic hearing loss, tinnitus and/or sudden hearing loss. It is envisaged that the sortilin antagonist herein disclosed may be particularly useful for the prevention or treatment of noise-induced hearing loss and ototoxicity-induced hearing loss.

Thus, in an embodiment, the sortilin antagonist for use according to the invention may disrupt interaction between a sortilin molecule and a pro-neurotrophin molecule, or disrupt the interaction between a sortilin molecule and a p75NTR molecule. Said sortilin molecule may be mature sortilin or a sortilin-related molecule such as SorCS1, SorCS2, SorCS3 or SorLA.

Preferably, the sortilin antagonist is a sortilin inhibitor. As used herein, the term "sortilin inhibitor" refers to a substance that binds to a sortilin protein, thereby preventing it from binding to a pro-neurotrophin and preventing the formation of the aforementioned trimeric complex, or resulting in the formation of a trimeric complex that is less active or inactive. The term "sortilin inhibitor" may also refer to a soluble fragment of sortilin, or sortilin-related molecule, which can bind competitively to biologically available pro-neurotrophins but prevent formation of an effective trimeric complex. As a result, the sortilin inhibitor results in an inhibition of receptor activation, which would otherwise occur as a result of ligand binding and/or allows for a shift in unbound/biologically available pro-neurotrophin, which in turn can act through a secondary route/receptor either in its pro- or mature form, specifically, but not limited to Trk family receptors.

Preferably, the sortilin antagonist of the present invention prevents the protein-protein interaction between a sortilin molecule and a pro-neurotrophin, further preventing the formation of the apoptotic trimeric complex usually formed between sortilin, pro-neurotrophin and the p75NTR receptor, or resulting in the formation of a low affinity trimeric complex, which is biologically less active or inactive or has minimal activity.

The term "sortilin antagonist" is intended to include any molecule which has the desired effect herein described. Accordingly, the sortilin antagonist may for example be a small molecule pharmaceutical compound or a larger molecule, such as a biologic. Examples of suitable larger molecule biologics include polypeptides, such as immunoglobulins (i.e. antibodies) or fragments thereof. As would be understood by a person of skill in the art, a small molecule would typically be produced by means of a chemical process whereas a biologic may be produced by means of a biological process, such as recombinant expression in a living cell or in cell free expression system. Where the sortilin antagonist is a small molecule compound, the sortilin antagonist may have a molecular weight of <2000 Da, preferably the sortilin antagonist has a molecular weight of <1000 Da.

In a preferred aspect of the invention, the sortilin antagonist is a compound of Formula (I): 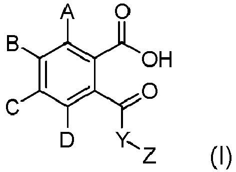 or a pharmaceutically acceptable salt, solvate, hydrate, geometrical isomer, tautomer, N-oxide, and/or prodrug thereof, wherein
Y is selected from the group consisting of -O-, -NR⁵-, and -S-;
Z is selected from the group consisting of optionally substituted C₁-C₆ alkyl, optionally substituted C₆-C₁₀ aryl, and optionally substituted C₁-C₉ heteroaryl;
A, B, C and D are each independently selected from the group consisting of H, optionally substituted C₁-C₆ alkyl, halo, NO₂, optionally substituted C₆-C₁₀ aryl, optionally substituted C₁-C₉ heteroaryl, -OR⁶, NR⁷R^{B}, -SR⁹, -C(O)OR¹⁰, -C(O)NR¹¹R¹², -C(O)SR¹³, C(O₂)SR¹⁴;
R¹, R⁴, R⁵, R⁶, R⁹, R¹⁰, R¹³, and R¹⁴ are each independently selected from the group consisting of H and C₁-C₆ alkyl group, wherein the C₁-C₆ alkyl is optionally substituted with one or more halo atoms; and
R², R³, R⁷, R⁸, R¹¹, and R¹² are each independently selected from the group consisting of H and C₁-C₆ alkyl, wherein the C₁-C₆ alkyl is optionally substituted with one or more halo atoms, or R² and R³, R⁷ and R⁸, and/or R¹¹ and R¹² can be taken together with the nitrogen atom to which they are attached to from a 5- or 6-membered heterocycle, optionally substituted with one or more halo atoms;
Compounds of Formula (I) act as sortilin antagonists by binding to sortilin. They are therefore useful in the treatment or prevention of diabetic retinopathy.
It is highly preferred that Y is -NR⁵-, more preferably Y is -NH-. In a particularly preferred aspect of the invention, the compound of Formula (I) is a compound of Formula (Ia):

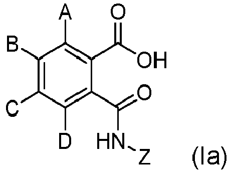

Preferred compounds of Formula (I) and (Ia), as detailed below, may exhibit increased binding affinity to sortilin and therefore increased efficacy in the treatment or prevention of hearing loss.

In a preferred aspect of the invention, A and D are H.

In another preferred aspect of the invention, B and C are independently selected from the group consisting of H, halo, CF₃, NO₂, and C₁-C₄ alkyl.

It is more preferred that C is H.

It is also more preferred that B is selected from the group consisting of halo, t-Bu, and CF₃, more preferably B is Br, I, t-Bu or CF₃.

In another preferred aspect of the invention, Z is an optionally substituted C₆-C₁₀ aryl or an optionally substituted C₁-C₉ heteroaryl.

It is preferred that the optionally substituted C₆-C₁₀ aryl is an optionally substituted phenyl.

Particularly preferred optionally substituted C₁-C₉ heteroaryl groups are optionally substituted 5- or 6-membered heteroaryl groups in which 1 to 5 of the ring atoms are carbon and one or more of the ring atoms are a heteroatom, more preferably optionally substituted 6-membered heteroaryl groups in which 1 to 5 of the ring atoms are carbon and one or more of the ring atoms are a heteroatom.

It is preferred that the ring atoms of the optionally substituted 6-membered heteroaryl group are selected from the group consisting of carbon and nitrogen. In which case, particularly preferred examples of optionally substituted C₁-C₉ heteroaryl groups are optionally substituted pyridyl, pyridazinyl, pyrimidinyl, pyrazinyl, and triazinyl. More preferred optionally substituted C₁-C₉ heteroaryl groups are optionally substituted pyridyl and pyrimidinyl.

Therefore, in a particularly preferred aspect of the invention, Z is an optionally substituted phenyl, an optionally substituted pyridyl, or an optionally substituted pyrimidinyl.

Z may be substituted with one or more substituents independently selected from the group consisting of -OR¹⁵, halo, and C₁-C₄ alkyl, wherein R¹⁵ is H, halo, and C₁-C₃ alkyl. More preferably, Z is substituted with one or more substituents selected from the group consisting of Cl, -OMe, and Me.

In addition to the above-mentioned preferred optional substituents, an alkylene group may be attached to two adjacent atoms of group Z to form a 5- or 6-membered partially saturated or saturated ring, optionally wherein the alkylene group is substituted with one or more halo atoms. Examples of such fused bicyclic ring systems are shown below in the following two examples of group Z:

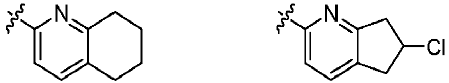

Particular combinations of Z and its substituents may be particularly preferred, as detailed below.

In a particularly preferred aspect of the invention, Z is C₆-C₁₀ aryl or C₁-C₉ heteroaryl. Each of C₆-C₁₀ aryl and C₁-C₉ heteroaryl are independently substituted with one or more substituents independently selected from the group consisting of -OR¹⁵, halo, and C₁-C₄ alkyl, wherein R¹⁵ is H, halo, or C₁-C₃ alkyl, preferably one or more substituents independently selected from the group consisting of Cl, -OMe, and Me; and/or an alkylene group is attached to two adjacent atoms of group Z to form a 5- or 6-membered partially saturated or saturated ring, optionally wherein the alkylene group is substituted with one or more halo atoms.

In an even more preferred aspect of the invention, Z is phenyl, pyridyl, or pyrimidinyl. Each of phenyl, pyridyl, and pyrimidinyl are independently substituted with one or more substituents independently selected from the group consisting of -OR¹⁵, halo, and C₁-C₄ alkyl, wherein R¹⁵ is H, halo, or C₁-C₃ alkyl, preferably one or more substituents independently selected from the group consisting of Cl, -OMe, and Me; and/or an alkylene group is attached to two adjacent atoms of group Z to form a 5- or 6-membered partially saturated or saturated ring, optionally wherein the alkylene group is substituted with one or more halo atoms.

Particular compounds of Formula (I) for use according to the invention are:
- 2-[(6-methylpyridin-2-yl)carbamoyl]benzoic acid;
- 2-methyl-6-[(6-methylpyridin-2-yl)carbamoyl]benzoic acid;
- 5-bromo-2-[(6-methylpyridin-2-yl)carbamoyl]benzoic acid;
- 5-chloro-2-[(6-methylpyridin-2-yl)carbamoyl]benzoic acid;
- 5-methyl-2-[(6-methylpyridin-2-yl)carbamoyl]benzoic acid;
- 2-[(6-methylpyridin-2-yl)carbamoyl]-5-(propan-2-yl)benzoic acid;
- 2-[(6-methylpyridin-2-yl)carbamoyl]-5-(trifluoromethyl)benzoic acid;
- 2-[(6-methylpyridin-2-yl)carbamoyl]-5-nitrobenzoic acid;
- 4-[(6-methylpyridin-2-yl)carbamoyl]-[1,1'-biphenyl]-3-carboxylic acid;
- 4-bromo-2-[(6-methylpyridin-2-yl)carbamoyl]benzoic acid;
- 4-chloro-2-[(6-methylpyridin-2-yl)carbamoyl]benzoic acid;
- 4-methyl-2-[(6-methylpyridin-2-yl)carbamoyl]benzoic acid;
- 2-[(6-methylpyridin-2-yl)carbamoyl]-4-(trifluoromethyl)benzoic acid;
- 4,5-dichloro-2-[(6-methylpyridin-2-yl)carbamoyl]benzoic acid;
- 4,5-dimethyl-2-[(6-methylpyridin-2-yl)carbamoyl]benzoic acid;
- 3-methyl-2-[(6-methylpyridin-2-yl)carbamoyl]benzoic acid;
- 5-bromo-2-[(butan-2-yl)carbamoyl]benzoic acid;
- 5-bromo-2-[(propan-2-yl)carbamoyl]benzoic acid;
- 5-bromo-2-(phenylcarbamoyl)benzoic acid;
- 5-bromo-2-[(3-methylphenyl)carbamoyl]benzoic acid;
- 5-bromo-2-[(pyridin-2-yl)carbamoyl]benzoic acid;
- 5-bromo-2-[(6-chloropyridin-2-yl)carbamoyl]benzoic acid;
- 5-bromo-2-[(4-methylpyridin-2-yl)carbamoyl]benzoic acid;
- 5-bromo-2-[(3-methylpyridin-2-yl)carbamoyl]benzoic acid;
- 5-bromo-2-[(6-methylpyridin-3-yl)carbamoyl]benzoic acid;
- 5-bromo-2-[(2-methylpyridin-4-yl)carbamoyl]benzoic acid;
- 5-bromo-2-[(2-methylpyrimidin-4-yl)carbamoyl]benzoic acid;
- 2-[(6-methoxypyridin-2-yl)carbamoyl]-5-(trifluoromethyl)benzoic acid;
- 2-[(5,6-dimethylpyridin-2-yl)carbamoyl]-5-(trifluoromethyl)benzoic acid;
- 2-[(5,6,7,8-tetrahydroquinolin-2-yl)carbamoyl]-5-(trifluoromethyl)benzoic acid, or a pharmaceutically acceptable salt, solvate, hydrate, tautomer, optical isomer, N-oxide, and/or prodrug thereof.

Particularly preferred compounds of Formula (I) for use according to the invention are:
- 5-bromo-2-[(6-methylpyridin-2-yl)carbamoyl]benzoic acid;
- 5-chloro-2-[(6-methylpyridin-2-yl)carbamoyl]benzoic acid;
- 5-methyl-2-[(6-methylpyridin-2-yl)carbamoyl]benzoic acid;
- 2-[(6-methylpyridin-2-yl)carbamoyl]-5-(propan-2-yl)benzoic acid;
- 2-[(6-methylpyridin-2-yl)carbamoyl]-5-(trifluoromethyl)benzoic acid;
- 2-[(6-methylpyridin-2-yl)carbamoyl]-5-nitrobenzoic acid;
- 4-bromo-2-[(6-methylpyridin-2-yl)carbamoyl]benzoic acid;
- 2-[(6-methylpyridin-2-yl)carbamoyl]-4-(trifluoromethyl)benzoic acid;
- 4,5-dichloro-2-[(6-methylpyridin-2-yl)carbamoyl]benzoic acid;
- 4,5-dimethyl-2-[(6-methylpyridin-2-yl)carbamoyl]benzoic acid;
- 5-bromo-2-[(3-methylphenyl)carbamoyl]benzoic acid;
- 5-bromo-2-[(pyridin-2-yl)carbamoyl]benzoic acid;
- 5-bromo-2-[(6-chloropyridin-2-yl)carbamoyl]benzoic acid;
- 5-bromo-2-[(4-methylpyridin-2-yl)carbamoyl]benzoic acid;
- 5-bromo-2-[(2-methylpyridin-4-yl)carbamoyl]benzoic acid;
- 5-bromo-2-[(2-methylpyrimidin-4-yl)carbamoyl]benzoic acid;
- 2-[(6-methoxypyridin-2-yl)carbamoyl]-5-(trifluoromethyl)benzoic acid;
- 2-[(5,6-dimethylpyridin-2-yl)carbamoyl]-5-(trifluoromethyl)benzoic acid;
- 2-[(5,6,7,8-tetrahydroquinolin-2-yl)carbamoyl]-5-(trifluoromethyl)benzoic acid;
or a pharmaceutically acceptable salt, solvate, hydrate, tautomer, optical isomer, N-oxide, and/or prodrug thereof.

In another preferred aspect of the invention, the sortilin antagonist is a compound of Formula (II): 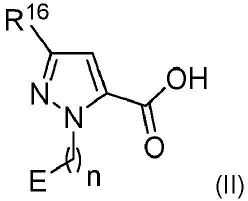 or a pharmaceutically acceptable salt, solvate, hydrate, tautomer, optical isomer, N-oxide, and/or prodrug thereof, wherein
R¹⁶ is C₁-C₆ alkyl, optionally substituted with one or more halo atoms;
n is 0, 1, or 2;
E is selected from the group consisting of H, optionally substituted 6- to 10-membered aryl, and optionally substituted 5- to 10-membered heteroaryl;
Preferred compounds of Formula (II), as detailed below, may exhibit increased binding affinity to sortilin and therefore increased efficacy in the treatment or prevention of hearing loss according to the invention.

It is preferred that R¹⁶ is t-butyl or neopentyl, most preferably t-butyl.

It is also preferred that n is 1 or 2, most preferably n is 1.

In preferred compounds of Formula (II), E is selected from the group consisting of H, optionally substituted phenyl, and optionally substituted 5- or 6-membered heteroaryl.

Preferably, phenyl and 5- or 6-membered heteroaryl are each independently optionally substituted with one or more substituents independently selected from the group consisting of:
(i) halo, C₁-C₃ alkyl, and C₁-C₃ alkoxy; and
(ii) phenyl, -O-phenyl, -CH₂-phenyl, 5- or 6-membered heteroaryl, -O-(5- or 6-membered heteroaryl), and -CH₂-(5- or 6-membered heteroaryl), each of which is independently optionally substituted with one or more halo atoms.

In a more preferred aspect of the invention, E is selected from the group consisting of:
(a) H;
(b) phenyl and 5- or 6-membered heteroaryl, optionally substituted with one or more substituents independently selected from the group consisting of halo, phenyl, phenoxyl, and 5- or 6-membered heteroaryl.

Preferably, each 5-membered heteroaryl group defined above is independently selected from the group consisting of pyrrolyl, pyrazolyl, imidazolyl, triazolyl, furanyl, oxazolyl, isoxazolyl, oxadiazolyl.

Preferably, each 6-membered heteroaryl group defined above is independently selected from the group consisting of pyridyl, pyrimidinyl, pyridazinyl, pyrazinyl, triazinyl, most preferably pyridyl.

In an even more preferred aspect of the invention, E is selected from the group consisting of:
(a) H;
(b) phenyl, optionally substituted with one or more halo atoms; and
(c) pyridyl and oxadiazolyl, optionally substituted with one or more substituents independently selected from halo, phenyl, phenoxyl, and pyrazolyl.

Particular compounds of Formula (II) for use according to the invention are:
- 1-benzyl-3-tert-butyl-1H-pyrazole-5-carboxylic acid;
- 3-tert-butyl-1-[(3,5-dichlorophenyl)methyl]-1H-pyrazole-5-carboxylic acid;
- 3-tert-butyl-1-{[6-(1H-pyrazol-1-yl)pyridin-3-yl]methyl}-1H-pyrazole-5-carboxylic acid;
- 3-tert-butyl-1-[(6-phenoxypyridin-3-yl)methyl]-1H-pyrazole-5-carboxylic acid;
- 3-tert-butyl-1-methyl-1H-pyrazole-5-carboxylic acid;
- 3-tert-butyl-1-phenyl-1H-pyrazole-5-carboxylic acid;
- 3-tert-butyl-1-(2-phenylethyl)-1H-pyrazole-5-carboxylic acid;
- 3-tert-butyl-1-[(5-phenyl-1,3,4-oxadiazol-2-yl)methyl]-1H-pyrazole-5-carboxylic acid;
- 1-benzyl-3-(2,2-dimethylpropyl)-1H-pyrazole-5-carboxylic acid;
- 1-(2-phenylethyl)-3-(propan-2-yl)-1H-pyrazole-5-carboxylic acid;
- 1-benzyl-3-(3,3-dimethylbutyl)-1H-pyrazole-5-carboxylic acid;
or a pharmaceutically acceptable salt, solvate, hydrate, tautomer, optical isomer, N-oxide, and/or prodrug thereof.

Particularly preferred compounds of Formula (II) for use according to the invention are:
- 1-benzyl-3-tert-butyl-1H-pyrazole-5-carboxylic acid;
- 3-tert-butyl-1-[(3,5-dichlorophenyl)methyl]-1H-pyrazole-5-carboxylic acid;
- 3-tert-butyl-1-[(6-phenoxypyridin-3-yl)methyl]-1H-pyrazole-5-carboxylic acid;
- 3-tert-butyl-1-[(5-phenyl-1,3,4-oxadiazol-2-yl)methyl]-1H-pyrazole-5-carboxylic acid;
or a pharmaceutically acceptable salt, solvate, hydrate, tautomer, optical isomer, N-oxide, and/or prodrug thereof.

In another preferred aspect of the invention, the sortilin antagonist is a compound of Formula (III) or (IV): 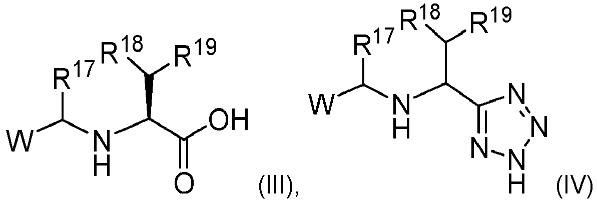 or a pharmaceutically acceptable salt, solvate, hydrate, geometrical isomer, tautomer, N-oxide, and/or prodrug thereof, wherein
W is selected from the group consisting of optionally substituted C₁-C₆ alkyl, optionally substituted 6- to 10-membered aryl, optionally substituted 5- to 10-membered heteroaryl, optionally substituted 3- to 7-membered cycloalkyl, and optionally substituted 5- or 6-membered heterocyclyl.
R¹⁷ is oxo or CF₃;
R¹⁸ is H or Me;
R¹⁹ is selected from the group consisting of C₁-C₆ alkyl, C₃-C₇ cycloalkyl, C₂-C₆ alkenyl, C₁-C₆ alkoxy, and phenyl.

Preferred compounds of Formula (III) or (IV), as detailed below, may exhibit increased binding affinity to sortilin and therefore increased efficacy in the treatment or prevention of hearing loss according to the invention.

It is preferred that R¹⁷ is oxo.

It is also preferred that R¹⁸ is H.

It is also preferred that R¹⁹ is selected from the group consisting of C₁-C₆ alkyl, C₃-C₇ cycloalkyl, C₂-C₆ alkenyl, and C₁-C₆ alkoxy, more preferably isopropyl, cyclohexyl, t-butyl, neopentyl, and t-butoxyl, more preferably t-butyl and neopentyl, most preferably neopentyl.

W is optionally substituted with one or more substituents. It is preferred that W is optionally substituted with one or more substituents independently selected from the group consisting of:
(i) halo, C₁-C₄ alkyl, and C₁-C₃ alkoxy; and
(ii) phenyl, -O-phenyl, -CH₂-phenyl, 5- or 6-membered heteroaryl, -O-(5- or 6-membered heteroaryl), and -CH₂-(5- or 6-membered heteroaryl), each of which is independently optionally substituted with one or more halo atoms;

In a more preferred aspect of the invention, W is selected from the group consisting of phenyl, -(C₁-C₄ alkyl)-phenyl, 5- or 6-membered heteroaryl, 5- or 6-membered heterocyclyl, C₃-C₇ cycloalkyl, and 9- or 10-membered bicyclic aryl or heteroaryl, each of which is independently optionally substituted with one or more substituents independently selected from the group consisting of:
(i) halo, C₁-C₄ alkyl, and C₁-C₃ alkoxy; and
(ii) benzyl and phenoxyl, each of which is independently optionally substituted with one or more halo atoms;

In an even more preferred aspect of the invention, W is selected from the group consisting of:
(a) phenyl and -(C₁-C₄ alkyl)-phenyl, wherein each phenyl group is optionally substituted with one or more substituents independently selected from halo and C₁-C₄ alkoxy;
(b) 5-membered heteroaryl, optionally substituted with one or more substituents independently selected from the group consisting of halo and C₁-C₄ alkyl;
(c) 6-membered heteroaryl and 6-membered heterocyclyl, optionally substituted with one or more substituents independently selected from the group consisting of halo, benzyl, and phenoxyl, wherein benzyl and phenoxyl are each independently optionally substituted with one or more halo atoms;
(d) C₃-C₇ cycloalkyl, optionally substituted with one or more halo atoms; and
(e) 10-membered bicyclic heteroaryl, optionally substituted with one or more halo atoms.

Preferably, the 5-membered heteroaryl of group W is selected from the group consisting of pyrrolyl, pyrazolyl, imidazolyl, triazolyl, furanyl, oxazolyl, isoxazolyl, oxadiazolyl, most preferably pyrrolyl and pyrazolyl.

Preferably, the 6-membered heteroaryl of group W is selected from the group consisting of pyridyl, pyrimidinyl, pyridazinyl, pyrazinyl, triazinyl, most preferably pyridyl.

Preferably, the 5-membered heterocyclyl of group W is selected from the group consisting of pyrrolidinyl, pyrrolinyl, pyrazolidinyl, imidazolidinyl, pyrazolinyl, imidazolinyl, tetrahydrofuranyl, and dioxolanyl.

Preferably, the 6-membered heterocyclyl of group W is selected from the group consisting of morpholinyl, piperidinyl, piperazinyl, dioxanyl, and tetrahydropyranyl, most preferably morpholinyl.

Preferably, the 9- or 10-membered bicyclic aryl of heteroaryl of group W is selected from the group consisting of indenyl, naphthalenyl, indolyl, isoindolyl, indazolyl, benzimidazolyl, azaindolyl, azaindazolyl, benzofuranyl, isobenzofuranyl, benzoisoxazolyl, benzoxazolyl, quinoxalinyl, phthalazinyl, cinnolinyl, quinazolinyl, naphthyridinyl, pyridopyrimidinyl, pyridopyrazinyl, quinolinyl, and isoquinolinyl, most preferably quinoxalinyl.

Preferably, the 10-membered bicyclic heteroaryl of group W is selected from the group consisting of quinoxalinyl, phthalazinyl, cinnolinyl, quinazolinyl, naphthyridinyl, pyridopyrimidinyl, pyridopyrazinyl, quinolinyl, isoquinolinyl, most preferably quinoxalinyl.

Particular compounds of Formula (III) and (IV) for use according to the invention are:
- (2S)-2-[(3,5-dichlorophenyl)formamido]-5,5-dimethylhexanoic acid;
- (2S)-2-[(4-chloro-1H-pyrrol-2-yl)formamido]-5,5-dimethylhexanoic acid;
- (2S)-5,5-dimethyl-2-[(6-phenoxypyridin-3-yl)formamido]hexanoic acid;
- (2S)-2-[(3,4-dichlorophenyl)formamido]-4-methylpentanoic acid;
- (2S)-2-[(3,5-dichlorophenyl)formamido]-4-methylpentanoic acid;
- (2S)-2-[(3-chlorophenyl)formamido]-4-methylpentanoic acid;
- (2S)-2-[(3-methoxyphenyl)formamido]-4-methylpentanoic acid;
- (2S)-2-{2-[(4-chlorophenyl)methyl]-2-methylpropanamido}-4-methylpentanoic acid;
- (2S)-4-methyl-2-[(1-methyl-1H-pyrazol-4-yl)formamido]pentanoic acid;
- (2S)-4-methyl-2-[(quinoxalin-2-yl)formamido]pentanoic acid;
- (2S)-2-(cyclopentylformamido)-4-methylpentanoic acid;
- (2S)-2-({4-[(3,5-dichlorophenyl)methyl]morpholin-2-yl}formamido)-4-methylpentanoic acid;
- (2S)-2-[(3,5-dichlorophenyl)formamido]-4-methylpent-4-enoic acid;
- (2S)-3-cyclopropyl-2-[(3,5-dichlorophenyl)formamido]propanoic acid;
- (2S)-3-cyclohexyl-2-[(3,5-dichlorophenyl)formamido]propanoic acid;
- (2S)-2-[(3,5-dichlorophenyl)formamido]-4,4-dimethylpentanoic acid;
- (2S)-3-{bicyclo[1.1.1]pentan-1-yl}-2-[(3,5-dichlorophenyl)formamido]propanoic acid;
- (2S)-2-[(3,5-dichlorophenyl)formamido]-3-phenylpropanoic acid;
- (2S)-3-(tert-butoxy)-2-[(3,5-dichlorophenyl)formamido]propanoic acid;
- (2S)-3-(tert-butoxy)-2-[(3,5-dichlorophenyl)formamido]butanoic acid;
- 3,5-dichloro-N-[3,3-dimethyl-1-(2H-1,2,3,4-tetrazol-5-yl)butyl]benzamide;
- (2S)-2-{[1-(3,5-dichlorophenyl)-2,2,2-trifluoroethyl]amino}-4-methylpentanoic acid;
- (2S)-4,4-dimethyl-2-[(6-phenoxypyridin-3-yl)formamido]pentanoic acid,
or a pharmaceutically acceptable salt, solvate, hydrate, tautomer, N-oxide, and/or prodrug thereof.

Particularly preferred compounds of Formula (III) and (IV) for use according to the invention are:
- (2S)-2-[(3,5-dichlorophenyl)formamido]-5,5-dimethylhexanoic acid;
- (2S)-2-[(4-chloro-1H-pyrrol-2-yl)formamido]-5,5-dimethylhexanoic acid;
- (2S)-5,5-dimethyl-2-[(6-phenoxypyridin-3-yl)formamido]hexanoic acid;
- (2S)-2-[(3,5-dichlorophenyl)formamido]-4-methylpentanoic acid;
- (2S)-2-({4-[(3,5-dichlorophenyl)methyl]morpholin-2-yl}formamido)-4-methylpentanoic acid;
- (2S)-3-cyclohexyl-2-[(3,5-dichlorophenyl)formamido]propanoic acid;
- (2S)-2-[(3,5-dichlorophenyl)formamido]-4,4-dimethylpentanoic acid;
- (2S)-3-(tert-butoxy)-2-[(3,5-dichlorophenyl)formamido]propanoic acid;
- (2S)-4,4-dimethyl-2-[(6-phenoxypyridin-3-yl)formamido]pentanoic acid;
or a pharmaceutically acceptable salt, solvate, hydrate, tautomer, N-oxide, and/or prodrug thereof.

According to another aspect of the invention, there is provided a pharmaceutical formulation comprising a sortilin antagonist according to the invention and one or more pharmaceutically acceptable carriers or excipients, for use in the treatment or prevention of hearing loss.

The compounds for use according to the invention may include isotopically-labelled and/or isotopically-enriched forms of the compounds. The compounds for use according to the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as ²H , ³H, ¹¹C, ¹³C, ¹⁴C, ¹³N, ¹⁵O, ¹⁷O, ³²P, ³⁵S, ¹⁸F, ³⁶Cl.

The compounds for use according to the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, geometrical isomers, tautomers, optical isomers, N-oxides, and/or prodrug forms thereof. It is to be understood that the compounds for use according to the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds for use according to the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2H- and 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereocenters). All stereoisomers, such as enantiomers and diastereomers, are intended unless otherwise indicated. Compounds for use according to the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds for use according to the present invention are described and may be isolated as a mixture of isomers or as separated isomeric forms.

In the case of the compounds, which contain an asymmetric carbon atom, the invention relates to the D form, the L form, and D, L mixtures and also, where more than one asymmetric carbon atom is present, to the diastereomeric forms. Those compounds for use according to the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

The term "prodrugs" refers to compounds that may be converted under physiological conditions or by solvolysis to a biologically active compound for use according to the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound for use according to the invention may be prepared by modifying functional groups, such as a hydroxy, amino or mercapto groups, present in a compound for use according to the invention in such a way that the modifications are cleaved, either in routine manipulation or in vivo, to the parent compound for use according to the invention. Examples of prodrugs include, but are not limited to, acetate, formate and succinate derivatives of hydroxy functional groups or phenyl carbamate derivatives of amino functional groups.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, is ELISA, radiolabeling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds for use according to the invention may be administered together with a physiologically acceptable carrier, excipient, and/or diluent (i.e. one, two, or all three of these). The pharmaceutical compositions disclosed herein may be administered by any suitable route, preferably by oral, ocular (including intravitreal), rectal, nasal, topical (including buccal and sublingual), sublingual, transdermal, intrathecal, transtympanic, transmucosal or parenteral (including subcutaneous, intramuscular, intravenous and intradermal) administration. Preferably, the pharmaceutical compositions are administered orally or transtympanically. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, etc. The formulations may be prepared by conventional methods in the dosage form of tablets, eye drops, creams, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow release formulations.

In a preferred embodiment, the sortilin antagonists herein described may be administered in a gel formulation, preferably a Poloxamer 407 gel formulation. Said formulation may comprise the following excipients, reactants and solvents; Poloxamer 407, 5% glucose, Sodium dihydrogenophosphate, Di-sodium hydrogenophosphate, acetonitrile, trifluoroacetic acid and de-ionized water. Preferably, the gel formulation is a 14 % poloxamer solution in diluted pH 7.4 phosphate buffer (0.013M) in deionized water. Such a formulation has been shown to be liquid (fluid) at room temperature and quickly becomes a gel at 37°C (body temperature), see Table 1 below. Additionally, the advantageous properties of such a formulation have been shown to be unaltered following the administration of the compounds herein disclosed, for example, AF38469 (example 8) at the targeted 1 mg/mL concentration. Accordingly, the aforementioned gel formulation is an appropriate vehicle solution to administer said compounds.

**Table 1: Poloxamer gelling tests at 20°C and 37°C.**

| Temperature | Time (min) | Poloxamer 407 | | | | |
|---|---|---|---|---|---|---|
| | | 13% | 14% | | 14.2% | 14.5% |
| | | 1 mL/vial | 1 mL/vial | 100µL/vial | 500µL/vial | 1 mL/vial |
| 20°C | T0 | fluid | fluid | fluid | fluid | fluid |
| 37°C | 2 | fluid | fluid | fluid | gel | gel |
| | 5 | fluid | fluid | very viscous | gel | gel |
| | 10 | fluid | slightly viscous | gel | gel | gel |
| | 15 | fluid | viscous | gel | gel | gel |
| | 20 | fluid | very viscous | gel | gel | gel |
| | 25 | fluid | flowing gel | gel | gel | gel |
| | 30 | fluid | gel | gel | gel | gel |

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

As described above, in addition to the small molecule compounds herein described, the sortilin antagonist of the present invention may also be a biologic molecule.

Accordingly, the sortilin antagonist for use according to the present invention may be an anti-sortilin antibody or an antigen-binding fragment thereof.

By the term "an antibody or antigen-binding fragment thereof" we intend any substantially intact antibody molecules, as well as chimeric antibodies, humanised antibodies, isolated human antibodies, single chain antibodies, bispecific antibodies, antibody heavy chains, antibody light chains, homodimers and heterodimers of antibody heavy and/or light chains, and antigen-binding fragments and derivatives of the same. Suitable antigen-binding fragments and derivatives include, but are not limited to, Fv fragments, Fab-like fragments, single variable domains and domain antibodies. The skilled person will recognise that using antibody fragments can confer a number of advantages compared to the use of whole antibodies. For example, the smaller size of the fragments may lead to improved pharmacological properties, such as better penetration of solid tissue. Moreover, antigen-binding fragments such as Fab, Fv, ScFv and dAb antibody fragments can be expressed in and secreted from bacteria, such as *E*. *coli,* thus allowing the facile production of large amounts of the said fragments.

The phrase "an antibody or an antigen-binding fragment thereof" is also intended to encompass antibody mimics (for example, non-antibody scaffold structures that have a high degree of stability yet allow variability to be introduced at certain positions). Those skilled in the art of biochemistry will be familiar with many such molecules, as discussed in Gebauer & Skerra, 2009, Curr Opin Chem Biol 13(3): 245-255 (the disclosures of which are incorporated herein by reference). Exemplary antibody mimics include: affibodies (also called Trinectins; Nygren, 2008, FEBS J, 275, 2668-2676); CTLDs (also called Tetranectins; Innovations Pharmac. Technol. (2006), 27-30); adnectins (also called monobodies; Meth. Mol. Biol., 352 (2007), 95-109); anticalins *(*Drug Discovery Today (2005), 10, 23-33); DARPins (ankyrins; Nat. Biotechnol. (2004), 22, 575-582); avimers (Nat. Biotechnol. (2005), 23, 1556-1561); microbodies (FEBS J, (2007), 274, 86-95); peptide aptamers (Expert. Opin. Biol. Ther. (2005), 5, 783-797); Kunitz domains (J. Pharmacol. Exp. Ther. (2006) 318, 803-809); affilins (Trends. Biotechnol. (2005), 23, 514-522); affimers (Avacta Life Sciences, Wetherby, UK).

The skilled person will appreciate that the invention also encompasses modified versions of the antibodies and antigen-binding fragments herein disclosed. For example, via the attachment of polyethylene glycol or another suitable polymer.

To elicit the desired effect, the sortilin antibody or the antigen-binding fragment herein disclosed may bind against any sequence of sortilin that produces said desired effect, i.e. an inhibitory effect. Such an effect may result from the inhibition of the binding of ligands to their respective binding sites, the inhibition of the formation of complexes with co-receptors or alterations in the confirmation of the receptor.

In preferred embodiments, the anti-sortilin antibody or the antigen-binding fragment thereof binds to at least one amino acid of any of the amino acid sequences according to SEQ ID NO: 1 to 14, or at least one amino acid of the amino acid sequence TGL.

The sortilin antibody or the antigen-binding fragment thereof may be a polyclonal antibody or a monoclonal antibody. Those skilled in the art will readily understand the methods of production by which polyclonal and monoclonal antibodies directed against sortilin are produced. The use of a polyclonal antibody may affect the binding of several ligands, due to polyclonal antibodies binding to several binding sites, accordingly several downstream pathways may be inhibited/disrupted. The use of a monoclonal antibody results in the antibody binding to a specific site and therefore will inhibit access of other molecules to that specific site.

The sortilin antibody or the antigen-binding fragment thereof may be obtained from a variety of different species. In a preferred embodiment, the sortilin antibody or antigen-binding fragment thereof may be a human anti-sortilin antibody, a goat anti-sortilin antibody, a rabbit anti-sortilin antibody or an IgG antibody. Said antibody or antigen-binding fragment thereof may also be a humanised antibody. Whilst the preferred antibody isotype may be an IgG antibody, it is understood that the present invention also encompasses other antibody isotypes, for example, IgM, IgD, IgA or IgE.

The sortilin antibody or the antigen-binding fragment thereof may also be conjugated to a cell-penetrating peptide in order to aid the delivery of the antibody or antigen-binding fragment to the desired location. Such a cell-penetrating peptide may be a TAT peptide.

### DEFINITIONS

"Optional" or "optionally" means that the subsequently described event or circumstance may, but need not, occur, and that the description includes instances where the event or circumstance occurs and instances in which it does not.

The term "C₁-C₆ alkyl" denotes a straight or branched alkyl group having from 1 to 6 carbon atoms, i.e. 1, 2, 3, 4, 5 or 6 carbon atoms. For parts of the range "C₁-C₆ alkyl" all subgroups thereof are contemplated, such as C₁-C₅ alkyl, C₁-C₄ alkyl, C₁-C₃ alkyl, C₁-C₂ alkyl, C₁ alkyl, C₂-C₆ alkyl, C₂-C₅ alkyl, C₂-C₄ alkyl, C₂-C₃ alkyl, C₂ alkyl, C₃-C₆alkyl, C₃-C₅alkyl, C₃-C₄alkyl, C₃alkyl, C₄-C₆alkyl, C₄-C₅alkyl, C₄alkyl, C₅-C₆ alkyl, C₅ alkyl, and C₆ alkyl. Examples of "C₁-C₆ alkyl" include methyl, ethyl, n-propyl, isopropyl, n-butyl, isobutyl, sec-butyl, and t-butyl.

When a term denotes a range, for instance "1 to 6 carbon atoms" in the definition of C₁-C₆ alkyl, each integer is considered to be disclosed, i.e. 1, 2, 3, 4, 5 and 6.

The term "C₃-C₇ cycloalkyl" denotes a cyclic or partially cyclic alkyl group having from 3 to 7 carbon atoms, i.e. 3, 4, 5, 6, or 7 carbon atoms. Examples of "C₃-C₇ cycloalkyl" include cyclopropyl, cyclobutyl, cyclopropylmethyl, cyclopentyl, and cyclohexyl. The term "cycloalkyl" includes bridged bicyclic cycloalkyl groups such as the following group:

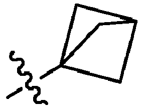

The term "C₂-C₆ alkenyl" denotes a straight or branched alkyl group having at least one carbon-carbon double bond, and having from 2 to 6 carbon atoms. Examples of "C₂-C₆ alkenyl" include 2-propenyl, 2-butenyl, 3-butenyl, 2-methyl-2-propenyl, 2-hexenyl, 5-hexenyl, 2,3-dimethyl-2-butenyl.

The term "C₁-C₆ alkoxy" denotes -O-(C₁-C₆ alkyl) in which a C₁-C₆ alkyl group is as defined above and is attached to the remainder of the compound through an oxygen atom. Examples of "C₁-C₆ alkoxy" include methoxy, ethoxy, n-propoxy, isopropoxy, n-butoxy, isobutoxy, sec-butoxy, t-butoxy and straight- and branched-chain pentoxy and hexoxy.

The term "halo atom" means a halogen atom, and is preferably F, Cl, Br or I.

The term "oxo" denotes a double bond to an oxygen atom (=O). This typically forms a ketone or aldehyde group.

The term "C₆-C₁₀ aryl" or "6- to 10-membered aryl" denotes an aromatic monocyclic or fused bicyclic hydrocarbon ring system comprising 6 to 10 ring atoms. Examples of "C₆-C₁₀ aryl" and "6- to 10-membered aryl" groups include phenyl, indenyl, naphthyl, and naphthalene.

The term "C₁-C₉ heteroaryl" or "5- to 10-membered heteroaryl" denotes an aromatic monocyclic or fused bicyclic heteroaromatic ring system having 5 to 10 ring atoms in which 1 to 9 of the ring atoms are carbon and one or more of the ring atoms are selected from nitrogen, sulphur, and oxygen. Examples of "C₁-C₉ heteroaryl" and "5- to 10-membered heteroaryl" include furyl, pyrrolyl, thienyl, oxazolyl, isoxazolyl, imidazolyl, thiazolyl, isothiazolyl, pyridinyl, pyrimidinyl, tetrazolyl, quinazolinyl, indolyl, indolinyl, isoindolyl, isoindolinyl, pyrazolyl, pyridazinyl, pyrazinyl, quinolinyl, quinoxalinyl, thiadiazolyl, benzofuranyl, 2,3-dihydrobenzofuranyl, 1,3-benzodioxolyl, 1,4-benzodioxinyl, 2,3-dihydro-1,4-benzodioxinyl, benzothiazolyl, benzimidazolyl, benzothiadiazolyl, benzotriazolyl and chromanyl.

The term "5-membered heterocycle" denotes a monocyclic ring system having 5 ring atoms in which 1 to 4 of the ring atoms are carbon and one or more of the ring atoms are selected from nitrogen, sulfur, and oxygen. Examples of "5-memebered heterocycle" include tetrahydrofuranyl, and pyrrolidinyl.

The term "6-membered heterocycle" denotes a monocyclic ring system having 6 ring atoms in which 1 to 5 of the ring atoms are carbon and one or more of the ring atoms are selected from nitrogen, sulfur, and oxygen. Examples of "5-memebered heterocycle" include tetrahydrofuranyl, and pyrrolidinyl.

"An effective amount" refers to an amount of a compound for use according to the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, ocular (such as intravitreal), topical, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds for use according to the invention may be disclosed by the name or chemical structure. If a discrepancy exists between the name of a compound and its associated chemical structure, then the chemical structure prevails.

### PREPARATION OF COMPOUNDS OF THE INVENTION

The compounds of Formulae (I), (II), (III), and (IV) disclosed herein may be prepared by, or in analogy with, conventional methods. Appropriate reaction conditions for the individual reaction steps are known to a person skilled in the art. The necessary starting materials for preparing the compounds of Formulae (I) and (II) are either commercially available, or may be prepared by methods known in the art.

The compounds of Formulae (I), (II), (III), and (IV) may possess one or more chiral carbon atoms, and they may therefore be obtained in the form of optical isomers, e.g., as a pure enantiomer, or as a mixture of enantiomers (racemate) or as a mixture containing diastereomers. The separation of mixtures of optical isomers to obtain pure enantiomers is well known in the art and may, for example, be achieved by fractional crystallization of salts with optically active (chiral) acids or by chromatographic separation on chiral columns.

Particular experimental procedures for examples of the invention are described below. The processes may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are mentioned above. The chemicals used in the synthetic routes delineated herein may include, for example, solvents, reagents, catalysts, and protecting group and deprotecting group reagents. Examples of protecting groups are t-butoxycarbonyl (Boc), benzyl and trityl(triphenylmethyl). The methods described below may also additionally include steps, either before or after the steps described specifically herein, to add or remove suitable protecting groups in order to ultimately allow synthesis of the compounds. In addition, various synthetic steps may be performed in an alternate sequence or order to give the desired compounds.

Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilize the present invention to its fullest extent. All references and publications cited herein are hereby incorporated by reference in their entirety.

### GENERAL SYNTHETIC PROCEDURE 1

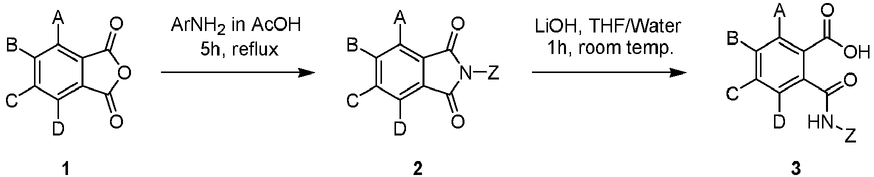

In general synthetic procedure 1, phthalic anhydride **1** undergoes condensation-*cyclo*-dehydartion to produce phthalimide **2** followed by hydrolysis to form phthalamic acid **3**. The regioisomers of phthalamic acid **3** are then separated via preparative H PLC.⁸

### GENEREAL SYNTHETIC PROCEDURE 2

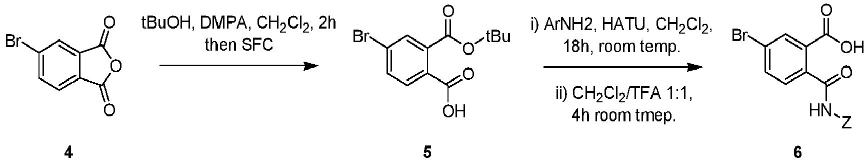

In general, synthetic procedure 2, phthalic anhydride **4** is reacted with *tert*-butanol followed by separation of the regioisomeric mono esters by supercritical fluid chromatography (SFC) to produce phthalate mono-*t*-butyl ester **5**. Phthalate mono-*t*-butyl ester **5** subsequently undergoes coupling deprotection resulting in phthalamic acid **6**.⁸

Palladium-catalysed coupling could be used to replace the bromine of phthalate mono-*t*-butyl ester **5** or phthalamic acid **6** with alternative substituents.⁸

### EXAMPLES

### Examples 1 and 2

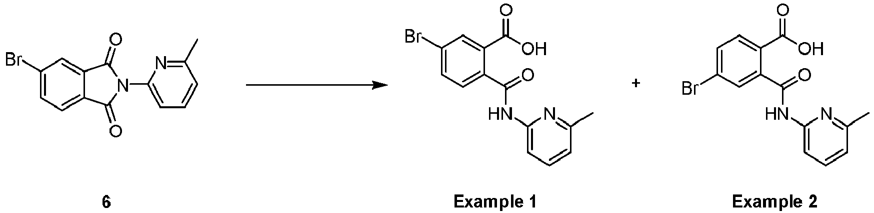

Phthalamide **6** was degraded under assay conditions (see description of Neurotensin (NTS) scintillation proximity assay (SPA) below) to afford the two corresponding phthalimide hydrolysis products, 5-bromo-2-[(6-methylpyridin-2-yl)carbamoyl]benzoic acid **(Example 1**) and 4-bromo-2-[(6-methylpyridin-2-yl)carbamoyl]benzoic acid **(Example 2**).⁸

Alternatively, Examples 1 and 2 may be prepared in accordance with general synthetic procedures 1 and/or 2.

### Examples 3 to 30

Examples 3 to 30 can be prepared in accordance with general synthetic procedures 1 and/or 2.

| **Ex.** | **Compound Name** | **Structure** |
|---|---|---|
| 3 | 2-[(6-methylpyridin-2-yl)carbamoyl]benzoic acid | 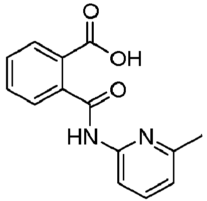 |
| 4 | 2-methyl-6-[(6-methylpyridin-2-yl)carbamoyl]benzoic acid | 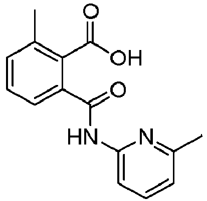 |
| 5 | 5-chloro-2-[(6-methylpyridin-2-yl)carbamoyl]benzoic acid | 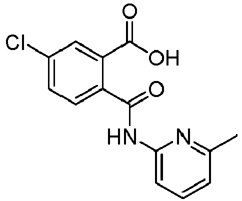 |
| 6 | 5-methyl-2-[(6-methylpyridin-2-yl)carbamoyl]benzoic acid | 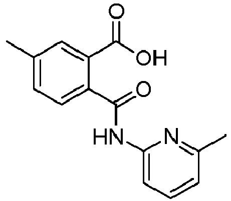 |
| 7 | 2-[(6-methylpyridin-2-yl)carbamoyl]-5-(propan-2-yl)benzoic acid | 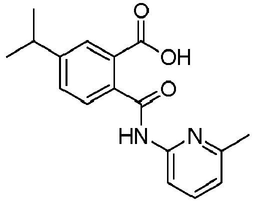 |
| 8 | 2-[(6-methylpyridin-2-yl)carbamoyl]-5-(trifluoromethyl)benzoic acid | 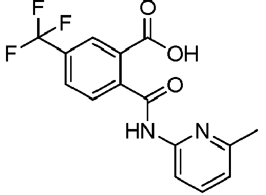 |
| 9 | 2-[(6-methylpyridin-2-yl)carbamoyl]-5-nitrobenzoic acid | 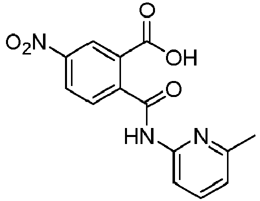 |
| 10 | 4-[(6-methylpyridin-2-yl)carbamoyl]-[1,1'-biphenyl]-3-carboxylic acid | 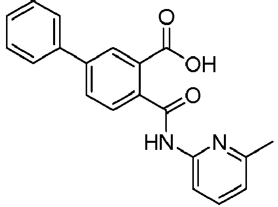 |
| 11 | 4-chloro-2-[(6-methylpyridin-2-yl)carbamoyl]benzoic acid | 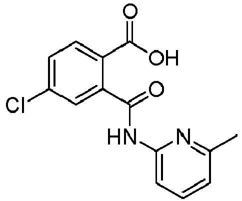 |
| 12 | 4-methyl-2-[(6-methylpyridin-2-yl)carbamoyl]benzoic acid | 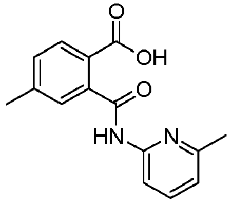 |
| 13 | 2-[(6-methylpyridin-2-yl)carbamoyl]-4-(trifluoromethyl)benzoic acid | 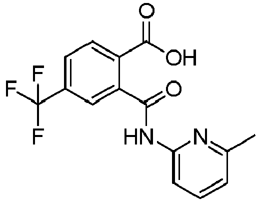 |
| 14 | 4,5-dichloro-2-[(6-methylpyridin-2-yl)carbamoyl]benzoic acid | 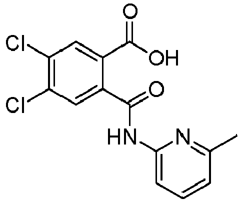 |
| 15 | 4,5-dimethyl-2-[(6-methylpyridin-2-yl)carbamoyl]benzoic acid | 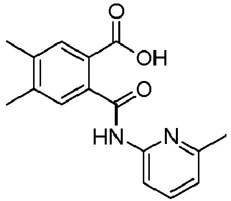 |
| 16 | 3-methyl-2-[(6-methylpyridin-2-yl)carbamoyl]benzoic acid | 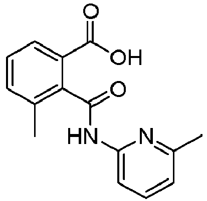 |
| 17 | 5-bromo-2-[(butan-2-yl)carbamoyl]benzoic acid | 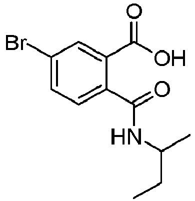 |
| 18 | 5-bromo-2-[(propan-2-yl)carbamoyl]benzoic acid | 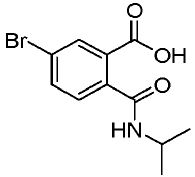 |
| 19 | 5-bromo-2-(phenylcarbamoyl)benzoic acid | 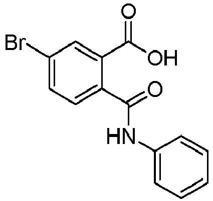 |
| 20 | 5-bromo-2-[(3-methylphenyl)carbamoyl]benzoic acid | 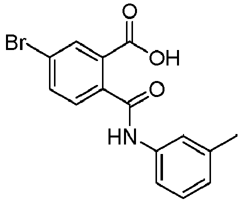 |
| 21 | 5-bromo-2-[(pyridin-2-yl)carbamoyl]benzoic acid | 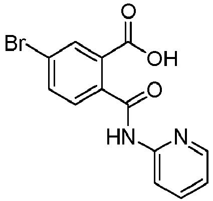 |
| 22 | 5-bromo-2-[(6-chloropyridin-2-yl)carbamoyl]benzoic acid | 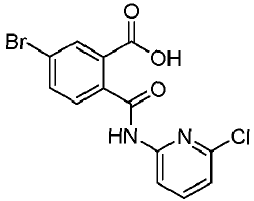 |
| 23 | 5-bromo-2-[(4-methylpyridin-2-yl)carbamoyl]benzoic acid | 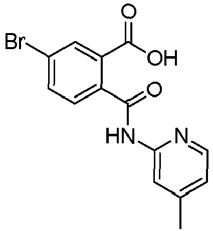 |
| 24 | 5-bromo-2-[(3-methylpyridin-2-yl)carbamoyl]benzoic acid | 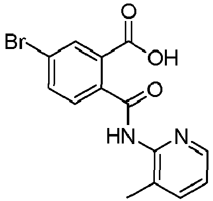 |
| 25 | 5-bromo-2-[(6-methylpyridin-3-yl)carbamoyl]benzoic acid | 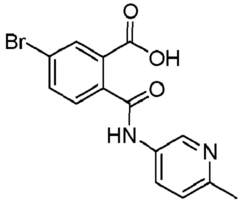 |
| 26 | 5-bromo-2-[(2-methylpyridin-4-yl)carbamoyl]benzoic acid | 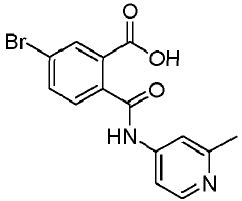 |
| 27 | 5-bromo-2-[(2-methylpyrimidin-4-yl)carbamoyl]benzoic acid | 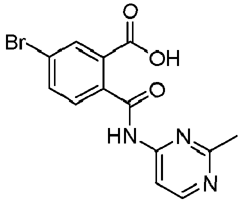 |
| 28 | 2-[(6-methoxypyridin-2-yl)carbamoyl]-5-(trifluoromethyl)benzoic acid | 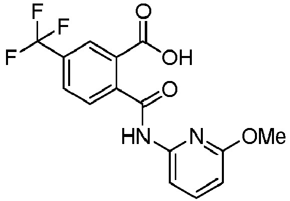 |
| 29 | 2-[(5,6-dimethylpyridin-2-yl)carbamoyl]-5-(trifluoromethyl)benzoic acid | 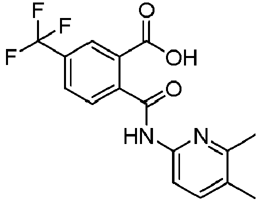 |
| 30 | 2-[(5,6,7,8-tetrahydroquinolin-2-yl)carbamoyl]-5-(trifluoromethyl)benzoic acid | 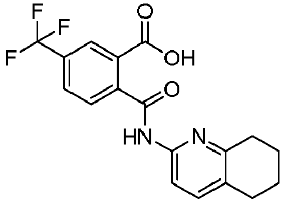 |

### Example 31

### 1-benzyl-3-tert-butyl-1H-pyrazole-5-carboxylic acid

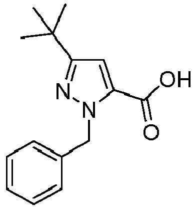

Ethyl 1-benzyl-3-(tert-butyl)-1H-pyrazole-5-carboxylate (0.1 g, 0.349 mmol) was dissolved in 1:1 THF/MeOH and heated to 50°C overnight. The reaction mixture was cooled, acidified using 1 N HCI and extracted 3 x EtOAc. The combined organic layers were dried over MgSO4, filtered and concentrated. Purification was performed using RP Gilson HPLC 5-95% 0.01% TFA-ACN over 20 min to provide the title compound (0.075 g, 83%). 1H NMR (500 MHz, Chloroform-d) δ 7.30-7.17 (m, 5H),6.83 (s, 1H), 5.71 (s, 2H), 1.34 (s, 9H). MS m/z: [M+H ]+ Calcd for C15H18N2O2 258.1; Found 260.3.⁹

### Examples 32 to 41

| **Ex.** | **Compound Name** | **Structure** |
|---|---|---|
| 32 | 3-tert-butyl-1-[(3,5-dichlorophenyl)methyl]-1 H-pyrazole-5-carboxylic acid | 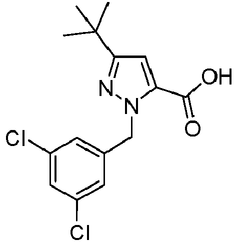 |
| 33 | 3-tert-butyl-1-{[6-(1 H-pyrazol-1 - yl)pyridin-3-yl]methyl}-1 H-pyrazole-5-carboxylic acid | 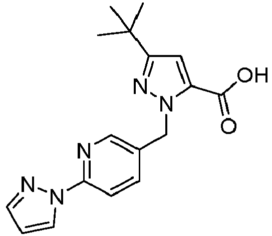 |
| 34 | 3-tert-butyl-1-[(6-phenoxypyridin-3-yl)methyl]-1 H-pyrazole-5-carboxylic acid | 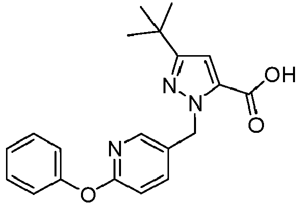 |
| 35 | 3-tert-butyl-1-methyl-1 H-pyrazole-5-carboxylic acid | 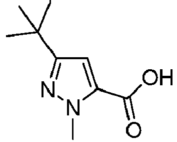 |
| 36 | 3-tert-butyl-1-phenyl-1 H-pyrazole-5-carboxylic acid | 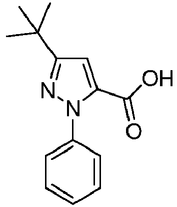 |
| 37 | 3-tert-butyl-1-(2-phenylethyl)-1 H-pyrazole-5-carboxylic acid | 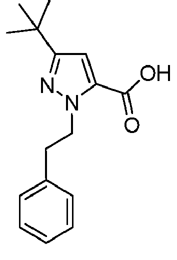 |
| 38 | 3-tert-butyl-1-[(5-phenyl-1,3,4-oxadiazol-2-yl)methyl]-1 H-pyrazole-5-carboxylic acid | 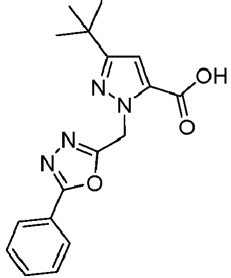 |
| 39 | 1-benzyl-3-(2,2-dimethylpropyl)-1 H-pyrazole-5-carboxylic acid | 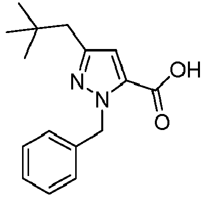 |
| 40 | 1-(2-phenylethyl)-3-(propan-2-yl)-1H-pyrazole-5-carboxylic acid | 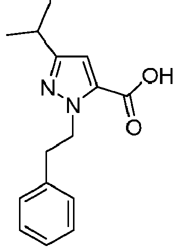 |
| 41 | 1-benzyl-3-(3,3-dimethylbutyl)-1 H-pyrazole-5-carboxylic acid | 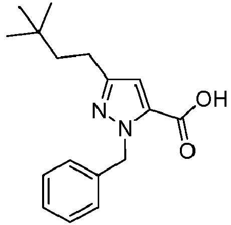 |

### Example 42

### (2S)-2-[(3,5-dichlorophenyl)formamido]-5,5-dimethylhexanoic acid

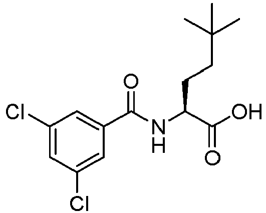

To a solution of 2-amino-5,5-dimethylhexanoic acid (racemic) (100 mg, 0.628 mmol) in tetrahydrofuran (3140 µl) was added 3,5-dichlorobenzoyl chloride (132 mg, 0.628 mmol) and 1N aqueous NaOH (1884 µl, 1.884 mmol). The resulting mixture was stirred overnight at room temperature. The reaction mixture was then concentrated and injected directly on to a reverse phase prep (5-95%MeCN/H2O TFA) providing 90 mg (43%) of a racemic white solid. The material was subsequently resolved using supercritical CO2 seperation on an IC (2 x 15 cm) column 15% isopropanol (0.1% DEA)/CO2 at 100 bar 70 mL/min. Chiral separation gave 48 mg of peak A (20.3%) and 49 mg of peak B (19.9%) with peak B as the desired product with > 99% ee. 1H NMR (500 MHz, Chloroform-d) δ 9.57 (s, 1H), 7.66 (d, J = 1.7 Hz, 2H), 7.50 (s, 1H), 6.76 (d, J = 7.7 Hz, 1H), 4.79 (q, J = 7.3 Hz, 1H), 2.08 - 1.90 (m, 1H), 1.89 - 1.73 (m, 1H), 1.29 (m, 2H), 0.89 (s, 9H). MS m/z: [M+H ]+ Calcd for C15H19Cl2NO3 331.07; Found 332.3.⁹

### Example 43

### (2S)-2-[(4-chloro-1H-pyrrol-2-yl)formamido]-5,5-dimethylhexanoic acid

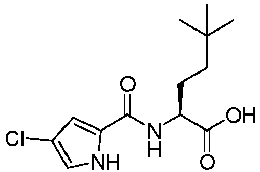

A vial was charged with 2-amino-5,5-dimethylhexanoic acid (250 mg, 1.570 mmol), 4-chloro-1H-pyrrole-2-carboxylic acid (286 mg, 1.963 mmol), DMF (5234 µl), DIEA (823 µl, 4.71 mmol), and HATU (746 mg, 1.963 mmol). The resulting mixture stirred was overnight at room temperature after which it was heated for 4 hours at 60°C. The reaction mixture was cooled to room temperature and purified by reverse phase prep (10-95%MeCN/H2O TFA) to provide 99.3 mg (22.1%) of racemic product as a brown solid. The material was subsequently resolved using supercritical CO2 separation on an AD-H (2 x 15 cm) column 15% isopropanol (0.1% DEA)/CO2 at 100 bar 60 mL/min. Chiral separation gave 43 mg of peak A (6.7%) and 41 mg of peak B (5.4%) with peak B as the desired product with > 99% ee. 1H NMR (500 MHz, DMSO-d6) δ 7.82 (s, 1H), 6.93 (s, 1H), 6.76 (s, 1H), 4.05 (s, 1H), 1.74 (m, 1H), 1.61 (m, 1H), 1.22 - 1.15 (m, 2H), 0.83 (s, 9H). MS m/z: [M+H ]+ Calcd for C13H19CIN2O3 286.1; Found 287.4.⁹

### Example 44

### (2S)-5,5-dimethyl-2-[(6-phenoxypyridin-3-yl)formamido]hexanoic acid

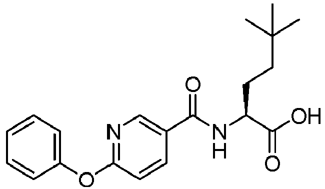

To a solution of 2-amino-5,5-dimethylhexanoic acid (100 mg, 0.628 mmol) in THF (3.1 mL) was added 6-phenoxynicotinoyl chloride (147 mg, 0.628 mmol) and 1 N NaOH (1884 µl, 1.884 mmol). The resulting mixture was stirred overnight at room temperature. The reaction mixture was concentrated and purified by reverse phase prep (10-95%MeCN/H2O TFA) to provide 22.5 mg (10.1%) of racemic product as a brown solid. The material was subsequently resolved using supercritical CO2 separation on an IA (2 x 15 cm) column 12% methanol (0.1% DEA)/CO2 at 100 bar 60 mL/min. Chiral separation gave 5.8 mg of peak A (2.6%) and 5.4 mg of peak B (2.4%) with peak B as the desired product with > 99% ee. 1H NMR (500 MHz, DMSO-d6) δ 12.62 (s, 1H), 8.67 (d, J = 7.6 Hz, 1H), 8.63 (d, J = 2.3 Hz, 1H), 8.29 (dd, J = 8.6, 2.4 Hz, 1H), 7.45 (t, J = 7.9 Hz, 2H), 7.25 (t, J = 7.4 Hz, 1H), 7.17 (d, J = 7.7 Hz, 2H), 7.11 (d, J = 8.5 Hz, 1H), 4.32 (s, 1H), 1.75 (dd, J = 32.9, 12.3 Hz, 2H), 1.41 - 1.13 (m, 2H), 0.87 (s, 9H). MS m/z: [M+H]+ Calcd for C20H24N2O4 356.2; Found 357.5.⁹

### Examples 45 to 64

| **Ex.** | **Compound Name** | **Structure** |
|---|---|---|
| 45 | 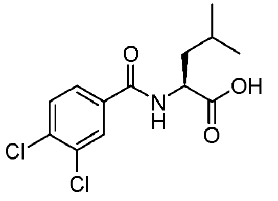 | (2S)-2-[(3,4-dichlorophenyl)formamido]-4-methylpentanoic acid |
| 46 | 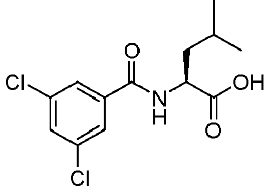 | (2S)-2-[(3,5-dichlorophenyl)formamido]-4-methylpentanoic acid |
| 47 | 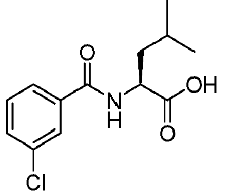 | (2S)-2-[(3-chlorophenyl)formamido]-4-methylpentanoic acid |
| 48 | 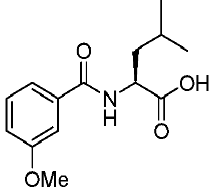 | (2S)-2-[(3-methoxyphenyl)formamido]-4-methylpentanoic acid |
| 49 | 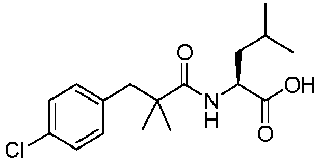 | (2S)-2-{2-[(4-chlorophenyl)methyl]-2-methylpropanamido}-4-methylpentanoic acid |
| 50 | 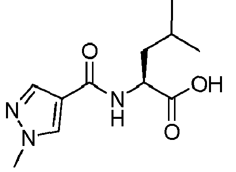 | (2S)-4-methyl-2-[(1-methyl-1 H-pyrazol-4-yl)formamido]pentanoic acid |
| 51 | 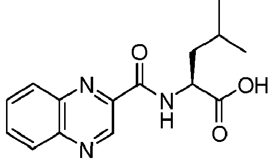 | (2S)-4-methyl-2-[(quinoxalin-2-yl)formamido]pentanoic acid |
| 52 | 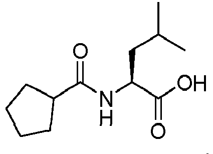 | (2S)-2-(cyclopentylformamido)-4-methylpentanoic acid |
| 53 | 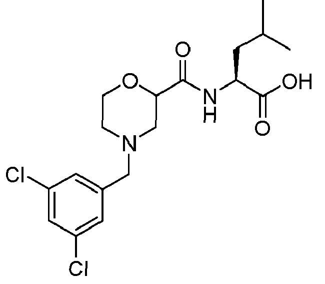 | (2S)-2-({4-[(3,5-dichlorophenyl)methyl]morpholin-2-yl}formamido)-4-methylpentanoic acid |
| 54 | 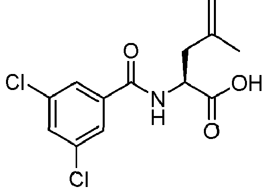 | (2S)-2-[(3,5-dichlorophenyl)formamido]-4-methylpent-4-enoic acid |
| 55 | 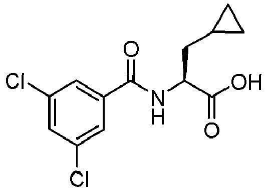 | (2S)-3-cyclopropyl-2-[(3,5-dichlorophenyl)formamido]propanoic acid |
| 56 | 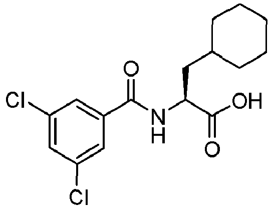 | (2S)-3-cyclohexyl-2-[(3,5-dichlorophenyl)formamido]propanoic acid |
| 57 | 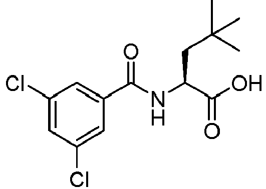 | (2S)-2-[(3,5-dichlorophenyl)formamido]-4,4-dimethylpentanoic acid |
| 58 | 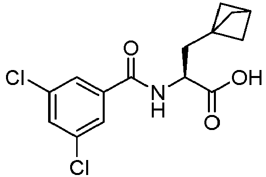 | (2S)-3-{bicyclo[1.1.1]pentan-1-yl}-2-[(3,5-dichlorophenyl)formamido]propanoic acid |
| 59 | 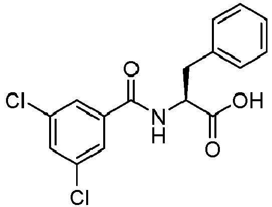 | (2S)-2-[(3,5-dichlorophenyl)formamido]-3-phenylpropanoic acid |
| 60 | 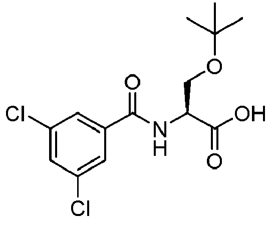 | (2S)-3-(tert-butoxy)-2-[(3,5-dichlorophenyl)formamido]propanoic acid |
| 61 | 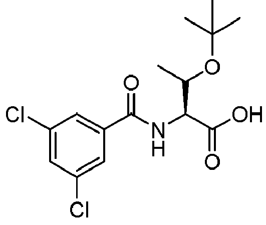 | (2S)-3-(tert-butoxy)-2-[(3,5-dichlorophenyl)formamido]butanoic acid |
| 62 | 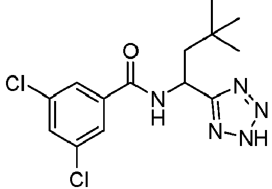 | 3,5-dichloro-N-[3,3-dimethyl-1-(2H-1,2,3,4-tetrazol-5-yl)butyl]benzamide |
| 63 | 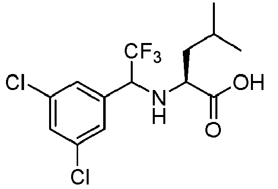 | (2S)-2-{[1-(3,5-dichlorophenyl)-2,2,2-trifluoroethyl]amino}-4-methylpentanoic acid |
| 64 | 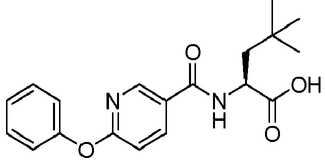 | (2S)-4,4-dimethyl-2-[(6-phenoxypyridin-3-yl)formamido]pentanoic acid |

### BIOLOGICAL DATA

### Neurotensin scintillation proximity assay

The exemplified compounds 1 to 30 of the invention were tested in a Neurotensin (NTS) scintillation proximity assay (SPA) and the IC₅₀ data is shown in the table below. NTS, which is a 13 amino acid neuropeptide, is a sortilin ligand. The IC₅₀ is a measure of the amount of the compound required to inhibit the binding of NTS to sortilin by 50%. The skilled person will recognise that the lower the IC₅₀ value, the less of the compound needed to achieve the desired effect, and as a result, the chances of undesirable off-target effects are reduced.

Compound affinity was determined by measuring the displacement of 3*H*-neurotensin binding to hSortilin in SPA format. Total volume of 40 µl in 50 mM HEPES pH 7.4 assay buffer containing 100 mM NaCl, 2.0 mM CaCI2, 0.1% BSA and 0.1% Tween-20. Compound pre-incubation for 30 min at RT with 150 nM of 6his-Sortilin before 5 nM [3*H*]-Neurotensin and Ni chelate imaging beads (Perkin Elmer) were added, after 6 h plate was read on a ViewLux with 360 s exposure time. Dose-response evaluation of compounds was performed with 10 concentrations of drugs (covering 3 decades). IC₅₀ values were calculated by nonlinear regression using the sigmoid concentration-response (variable slope) using Xlfit 4 (IDBS, UK). All values reported are average of at least 4 determinations.⁸

The data in the table below shows that the compounds disclosed herein are sortilin inhibitors.

| **Example** | **NTS IC₅₀ (nM)** | **Example** | **NTS IC₅₀ (nM)** |
|---|---|---|---|
| 1 | 710 | 16 | 43%* |
| 2 | 12000 | 17 | 30%* |
| 3 | 15%* | 18 | 35%* |
| 4 | 5%* | 19 | 69%* |
| 5 | 1400 | 20 | 5200 |
| 6 | 6400 | 21 | 3600 |
| 7 | 1100 | 22 | 1600 |
| 8 | 330 | 23 | 2400 |
| 9 | 1700 | 24 | 64%* |
| 10 | 2%* | 25 | 70%* |
| 11 | 69%* | 26 | 1800 |
| 12 | 57%* | 27 | 490 |
| 13 | 9100 | 28 | 1300 |
| 14 | 830 | 29 | 170 |
| 15 | 3400 | 30 | 100 |

| | | | |
|---|---|---|---|
| * % inhibition at 50 µM. | | | |

The exemplified compounds 31 to 64 of the invention were tested in a progranulin (PGRN) peptide/SORT homogenous time-resolved fluorescence (HTRF) assay and the IC₅₀ data shown in the table below. To determine the IC₅₀s of compounds bound to SORT and thus preventing its interaction with the biotinylated 18-mer PGRN peptide a time-resolved fluorescence resonance energy transfer (TR-FRET) format was used. All test compounds were dissolved in DMSO; the final concentration of DMSO in the reaction buffer was 1%. Compounds were prepared in a ten-point, three-fold serial dilution scheme using an acoustic dispenser. A solution of SORT protein in assay buffer (10 mM HEPES, pH 7.3, 150 mM NaCl, 0.005% Brij-35 and 0.01 % BSA, final concentration of SORT 1 nM) was added to each well and pre-incubated with compounds or DMSO vehicle at ambient temperature for 10 min with gentle shaking. Next, biotinylated 18-mer PGRN peptide was added to each well (final concentration 4 nM) and the binding reactions were incubated at ambient temperature for 30 min with gentle shaking. Solutions of SA/Dylight in assay buffer were added to each well, immediately followed by addition of Eu/anti-His in assay buffer. Reaction mixtures were incubated at ambient temperature for 120 min with gentle shaking, and the TR-FRET signal (the ratio of the 665 nm (Dylight) and 615 nm (Eu) fluorescence emissions using a 320 nm excitation wavelength) of each binding reaction was measured on an Envision plate reader (PerkinElmer) equipped with a LANCE/DELFIA Dual Enhanced mirror, a UV2(TRF) 320 nm excitation filter and APC 665 nm and Europium 615 nm emission filters, using the LANCE Eu/APC Dual 662 factory default protocol. Data were analyzed using ABase and a 4-parameter logistic fit based on the Levenberg-Marquardt algorithm.⁹

| **Example** | **IC₅₀ (µM)** | **Example** | **IC₅₀ (µM)** |
|---|---|---|---|
| 31 | 2.0 | 48 | 6.8 |
| 32 | 2.1 | 49 | 6.8 |
| 33 | 3.0 | 50 | 5.3 |
| 34 | 2.1 | 51 | 6.3 |
| 35 | 8.3 | 52 | 25.3 |
| 36 | 7.6 | 53 | 14.4 |
| 37 | 5.3 | 54 | 17.1 |
| 38 | 0.49 | 55 | 10.7 |
| 39 | 6.6 | 56 | 2.3 |
| 40 | >67 | 57 | 0.35 |
| 41 | >67 | 58 | 18 |
| 42 | 0.17 | 59 | >67 |
| 43 | 0.09 | 60 | 1.5 |
| 44 | 0.02 | 61 | >67 |
| 45 | 4.3 | 62 | 6.9 |
| 46 | 2.7 | 63 | 10.8 |
| 47 | 4.5 | 64 | 0.065 |

The below IC₅₀ data was obtained as described above for exemplified compounds 1 to 30, with the exception that the neurotensin employed in the SPA assay is [125I]-Neurotensin.

| **Compound** | **Expected IC₅₀ (µM)** | **Observed IC₅₀ (µM)** | **Fold difference between calculated and expected** |
|---|---|---|---|
| Example 8 | 0.316 | 0.460 | 1.4 |
| *Example 44 | 0.020 | 0.120 | 6 |
| Neurotensin | 0.410 | 0.626 | 1.5 |

| | | | |
|---|---|---|---|
| *Racemate | | | |

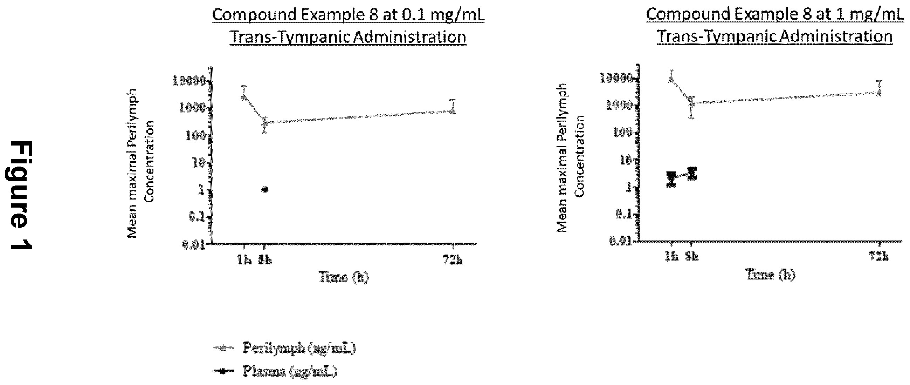

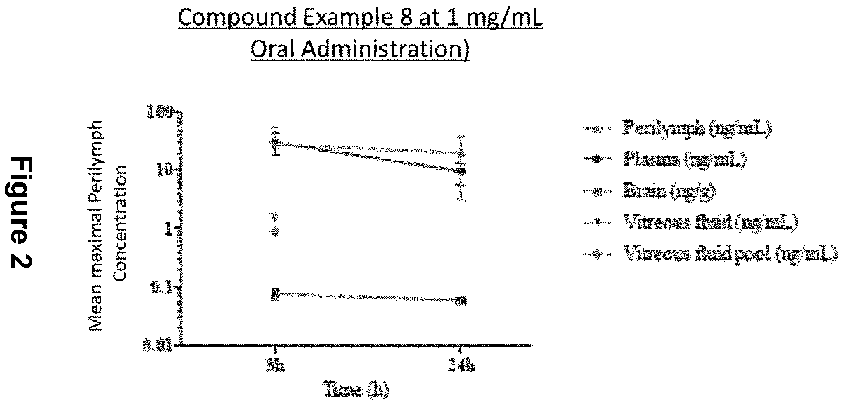

As can be seen from Figure 1, the compound according to Example 8 was present in the perilymph of male Hartley guinea-pigs up to the last time point of 72 hours following local dosing via the trans-tympanic route. This was evident at both doses tested (5 or 50 µg/ear; i.e. 0.1 or 1 mg/ml). Figure 2 also demonstrates that the compound according to Example 8 when administered orally (1 mg/mL) was also present in the perilymph of male Hartley guinea-pigs at the two time points tested (8 hours and 24 hours) following three days of daily dosing. The above data demonstrates how the compounds herein disclosed may be desirable for the prevention and/or treatment of hearing loss.

### References

1. Tauris, J., et al., Proneurotrophin-3 May Induce Sortilin-Dependent Death In Inner Ear Neurons. Eur J Neuroscience (2020), 33(4), pp.622-31.
2. Goettsch, C., et al., Sortilin and Its Multiple Roles in Cardiovascular and Metabolic Diseases. Atherosclerosis, Thrombosis and Vascular Biology (2017), 38(1), pp. 19-25.
3. Willnow, T.E., et al., Sortilins: new players in lipoprotein metabolism. Current Opinion in Lipidology (2011), 22(2), pp. 79-85.
4. Kjolby, M., et al., Sort1, encoded by the cardiovascular risk locaus 1p13.3, is a regulator of hepatic lipoprotein export. Cell Metabolism (2010), 12(3), pp. 213-223.
5. Jansen, P., et al., Roles for the pro-neurotrophin receptor sortilin in neuronal development, aging and brain injury. Nature Neuroscience (2007), 10(11), pp.1449-1457.
6. Tenk, H.K., et al., ProBDNF induces neuronal apoptosis via activation of a receptor complex of p75NTR and sortilin. J Neuroscience (2005), 10(11), pp.1449-1457.
7. Nykjaer, A., et al., Sortilin is essential for proNGF-induced neuronal cell death. Nature (2004), 427(6977), pp.843-848.
8. T. J. Schrøder et al. The identification of AF38469: An orally bioavailable inhibitor of the VPS10P family sorting receptor Sortilin, Bioorganic & Medicinal Chemistry Letters 24 (2014) 177-180.
9. Shawn J. Stachel, et al., Identification of potent inhibitors of the sortilin-progranulin interaction, Bioorganic & Medicinal Chemistry Letters, 30 (2020).

### Sequences referenced throughout the specification and forming part of the description.

SEQ ID NO: 1 (full length sortilin- isoform 1)

1 
51 
101 
151 
201 
251 
301 
351 
401 
451 
501 
551 
601 
651 
701 
751 
801 

SEQ ID NO: 2 (full length sortilin- isoform 2)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT
601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE

SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI

361 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

SEQ ID NO: 4 (Extracellular domain of sortilin)

1 QDRLDAPPP PAAPLPRWSG PIGVSWGLRA AAAGGAFPRG GRWRRSAPGE
51 DEECGRVRDF VAKLANNTHQ HVFDDLRGSV SLSWVGDSTG VILVLTTFHV
101 PLVIMTFGQS KLYRSEDYGK NFKDITDLIN NTFIRTEFGM AIGPENSGKV
151 VLTAEVSGGS RGGRIFRSSD FAKNFVQTDL PFHPLTQMMY SPQNSDYLLA
201 LSTENGLWVS KNFGGKWEEI HKAVCLAKWG SDNTIFFTTY ANGSCKADLG
251 ALELWRTSDL GKSFKTIGVK IYSFGLGGRF LFASVMADKD TTRRIHVSTD
301 QGDTWSMAQL PSVGQEQFYS ILAANDDMVF MHVDEPGDTG FGTIFTSDDR
351 GIVYSKSLDR HLYTTTGGET DFTNVTSLRG VYITSVLSED NSIQTMITFD
401 QGGRWTHLRK PENSECDATA KNKNECSLHI HASYSISQKL NVPMAPLSEP
451 NAVGIVIAHG SVGDAISVMV PDVYISDDGG YSWTKMLEGP HYYTILDSGG
501 IIVAIEHSSR PINVIKFSTD EGQCWQTYTF TRDPIYFTGL ASEPGARSMN
551 ISIWGFTESF LTSQWVSYTI DFKDILERNC EEKDYTIWLA HSTDPEDYED
601 GCILGYKEQF LRLRKSSVCQ NGRDYVVTKQ PSICLCSLED FLCDFGYYRP
651 ENDSKCVEQP ELKGHDLEFC LYGREEHLTT NGYRKIPGDK CQGGVNPVRE
701 VKDLKKKCTS NFLSPEKQNS KSNS

SEQ ID NO: 5 (Neurotensin binding site 1)
   1 SVMADKDTTR RIHVSTDQGD TWSMAQLPSV GQEQFY
SEQ ID NO: 6 (Neurotensin binding site 2)
   1 GSVSL
SEQ ID NO: 7 (Binding site for the propeptide of sortilin)
   1 SISQKLNVPM APLSEPNAVG IVIAHGSVG
SEQ ID NO: 8 (proNGF binding site 1)
   1 RIFRSSDFAK NF
SEQ ID NO: 9 (proNGF binding site 2)
   1 SVMADKDTTR RIHVSTDQGD TWSMAQLPSV GQEQFY
SEQ ID NO: 10 (proNGF binding site 3)
   1 SEDNSIQTM
SEQ ID NO: 11 (proNGF binding site 4)
   1 IHASI
SEQ ID NO: 12 (proNGF binding site 5)
   1 VIAHGSVGDA IS
SEQ ID NO: 13 (proNGF binding site 6)
   1 GYRKIPGDKC QGGVNPV

SEQ ID NO: 14 (NGF binding site)

1 CEEKDYTIWL AHSTDPEDYED GCILGYKEQF LRLRKSSVCQ NGRDYVVTKQ
51 PSICLCSLED FLCDFGYYRP ENDSKCVEQP ELKGHDLEFC LYGREEHLTT
101 NGYRKIPGDK CQGGVNPVRE VKDLKKKCTS NFLSPEKQNS KSNS

## Claims

1. A sortilin antagonist for use in the prevention or treatment of hearing loss.

2. The sortilin antagonist for use according to claim 1, wherein the hearing loss is noise-induced hearing loss, ototoxicity-induced hearing loss, age-induced hearing loss, idiopathic hearing loss, tinnitus or sudden hearing loss.

3. The sortilin antagonist for use according to claim 1 or 2, wherein
(i) the sortilin antagonist disrupts an interaction between a sortilin or sortilin-related molecule and a pro-neurotrophin molecule; and/or
(ii) the sortilin antagonist disrupts an interaction between a sortilin or sortilin-related molecule and a p75NTR molecule;
preferably, wherein the sortilin is mature sortilin or wherein the sortilin-related molecule is SorSC1, SorCS2, SorCS3 or SorLa.

4. The sortilin antagonist for use according to any one of claims 1 to 3, wherein the sortilin antagonist is a sortilin inhibitor.

5. The sortilin antagonist for use according to any one of claims 1 to 4, wherein the sortilin antagonist is a compound of Formula (I), (II), (III) or (IV), or a pharmaceutically acceptable salt, solvate, hydrate, geometrical isomer, tautomer, N-oxide, and/or prodrug thereof, 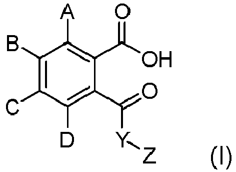
Y is selected from the group consisting of -O-, -NR⁵-, and -S-;
Z is selected from the group consisting of optionally substituted C₁-C₆ alkyl, optionally substituted C₆-C₁₀ aryl, and optionally substituted C₁-C₉ heteroaryl;
A, B, C and D are each independently selected from the group consisting of H, optionally substituted C₁-C₆ alkyl, halo, NO₂, optionally substituted C₆-C₁₀ aryl, optionally substituted C₁-C₉ heteroaryl, -OR⁶, NR⁷R⁸, -SR⁹, -C(O)OR¹⁰, - C(O)NR¹¹R¹², -C(O)SR¹³, C(O₂)SR¹⁴;
R¹, R⁴, R⁵, R⁶, R⁹, R¹⁰, R¹³, and R¹⁴ are each independently selected from the group consisting of H and C₁-C₆ alkyl group, wherein the C₁-C₆ alkyl is optionally substituted with one or more halo atoms; and
R², R³, R⁷, R⁸, R¹¹, and R¹² are each independently selected from the group consisting of H and C₁-C₆ alkyl, wherein the C₁-C₆ alkyl is optionally substituted with one or more halo atoms, or R² and R³, R⁷ and R⁸, and/or R¹¹ and R¹² can be taken together with the nitrogen atom to which they are attached to from a 5- or 6- membered heterocycle, optionally substituted with one or more halo atoms; 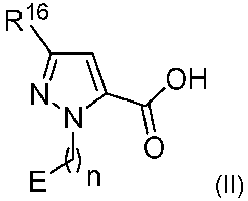
R¹⁶ is C₁-C₆ alkyl, optionally substituted with one or more halo atoms, preferably R¹⁶ is t-butyl or neopentyl, most preferably t-butyl;
n is 0, 1, or 2, preferably n is 1 or 2, most preferably n is 1;
E is selected from the group consisting of H, optionally substituted 6- to 10-membered aryl, and optionally substituted 5- to 10-membered heteroaryl; 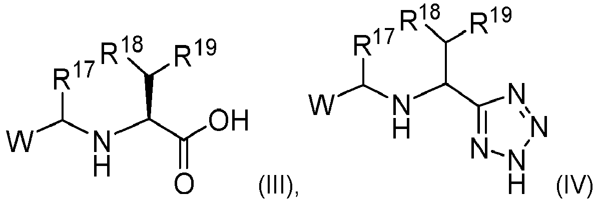
W is selected from the group consisting of optionally substituted C₁-C₆ alkyl, optionally substituted 6- to 10-membered aryl, optionally substituted 5- to 10-membered heteroaryl, optionally substituted 3- to 7-membered cycloalkyl, and optionally substituted 5- or 6-membered heterocyclyl, preferably wherein W is optionally substituted with one or more substituents independently selected from the group consisting of:
(i) halo, C₁-C₄ alkyl, and C₁-C₃ alkoxy; and
(ii) phenyl, -O-phenyl, -CH2-phenyl, 5- or 6-membered heteroaryl, -O-(5- or 6-membered heteroaryl), and -CH₂-(5- or 6-membered heteroaryl), each of which is independently optionally substituted with one or more halo atoms;
R¹⁷ is oxo or CF₃, preferably oxo;
R¹⁸ is H or Me, preferably H;
R¹⁹ is selected from the group consisting of C₁-C₆ alkyl, C₃-C₇ cycloalkyl, C₂-C₆ alkenyl, C₁-C₆ alkoxy, and phenyl, preferably C₁-C₆ alkyl, C₃-C₇ cycloalkyl, C₂-C₆ alkenyl, and C₁-C₆ alkoxy, more preferably isopropyl, cyclohexyl, t-butyl, neopentyl, and t-butoxyl, more preferably t-butyl and neopentyl, most preferably neopentyl.

6. The sortilin antagonist for use according to claim 5, wherein Y is -NR⁵-, preferably Y is -NH-.

7. The sortilin antagonist for use according to claim 5 or 6, wherein A and D are H; and/or
wherein B and C are independently selected from the group consisting of H, halo, CF₃, NO₂, and C₁-C₄ alkyl.

8. The sortilin antagonist for use according to any one of claims 5 to 7, wherein C is H; and/or
wherein B is selected from the group consisting of halo, t-Bu and CF₃, preferably wherein B is Br, I, t-Bu or CF₃.

9. The sortilin antagonist for use according to any one of claims 5 to 8, wherein Z is an optionally substituted C₆-C₁₀ aryl or an optionally substituted C₁-C₉ heteroaryl, more preferably Z is an optionally substituted phenyl, an optionally substituted pyridyl, or an optionally substituted pyrimidinyl.

10. The sortilin antagonist for use according to claim 9, wherein Z is substituted with one or more substituents independently selected from the group consisting of -OR¹⁵, halo, and C₁-C₄ alkyl, wherein R¹⁵ is H, halo, or C₁-C₃ alkyl, preferably wherein Z is substituted with one or more substituents selected from the group consisting of Cl, -OMe, and Me;
and/or wherein an alkylene group is attached to two adjacent atoms of group Z to form a 5- or 6-membered partially saturated or saturated ring, optionally wherein the alkylene group is substituted with one or more halo atoms.

11. The sortilin antagonist for use according to claim 5, wherein E is selected from the group consisting of H, phenyl, and 5- or 6-membered heteroaryl, wherein phenyl and 5- or 6-membered heteroaryl are each independently optionally substituted with one or more substituents independently selected from the group consisting of:
(i) halo, C₁-C₃ alkyl, and C₁-C₃ alkoxy; and
(ii) phenyl, -O-phenyl, -CH2-phenyl, 5- or 6-membered heteroaryl, -O-(5- or 6-membered heteroaryl), and -CH₂-(5- or 6-membered heteroaryl), each of which is independently optionally substituted with one or more halo atoms,
preferably E is selected from the group consisting of:
(a) H;
(b) phenyl and 5- or 6-membered heteroaryl, optionally substituted with one or more substituents independently selected from the group consisting of halo, phenyl, phenoxyl, and 5- or 6-membered heteroaryl.
more preferably E is selected from the group consisting of:
(a) H;
(b) phenyl, optionally substituted with one or more halo atoms; and
(c) pyridyl and oxadiazolyl, optionally substituted with one or more substituents independently selected from halo, phenyl, phenoxyl, and pyrazolyl.

12. The sortilin antagonist for use according to claim 5, wherein W is selected from the group consisting of phenyl, -(C₁-C₄ alkyl)-phenyl, 5- or 6-membered heteroaryl, 5- or 6-membered heterocyclyl, C₃-C₇ cycloalkyl, and 9- or 10-membered bicyclic aryl or heteroaryl, each of which is independently optionally substituted with one or more substituents independently selected from the group consisting of:
(i) halo, C₁-C₄ alkyl, and C₁-C₃ alkoxy; and
(ii) benzyl and phenoxyl, each of which is independently optionally substituted with one or more halo atoms;
preferably W is selected from the group consisting of:
(a) phenyl and -(C₁-C₄ alkyl)-phenyl, wherein each phenyl group is optionally substituted with one or more substituents independently selected from halo and C₁-C₄ alkoxy;
(b) 5-membered heteroaryl, optionally substituted with one or more substituents independently selected from the group consisting of halo and C₁-C₄ alkyl;
(c) 6-membered heteroaryl and 6-membered heterocyclyl, optionally substituted with one or more substituents independently selected from the group consisting of halo, benzyl, and phenoxyl, wherein benzyl and phenoxyl are each independently optionally substituted with one or more halo atoms;
(d) C₃-C₇ cycloalkyl, optionally substituted with one or more halo atoms; and
(e) 10-membered bicyclic heteroaryl, optionally substituted with one or more halo atoms.

13. The sortilin antagonist for use according to any preceding claim, wherein the sortilin antagonist is
• 2-[(6-methylpyridin-2-yl)carbamoyl]benzoic acid;
• 2-methyl-6-[(6-methylpyridin-2-yl)carbamoyl]benzoic acid;
• 5-bromo-2-[(6-methylpyridin-2-yl)carbamoyl]benzoic acid;
• 5-chloro-2-[(6-methylpyridin-2-yl)carbamoyl]benzoic acid;
• 5-methyl-2-[(6-methylpyridin-2-yl)carbamoyl]benzoic acid;
• 2-[(6-methylpyridin-2-yl)carbamoyl]-5-(propan-2-yl)benzoic acid;
• 2-[(6-methylpyridin-2-yl)carbamoyl]-5-(trifluoromethyl)benzoic acid;
• 2-[(6-methylpyridin-2-yl)carbamoyl]-5-nitrobenzoic acid;
• 4-[(6-methylpyridin-2-yl)carbamoyl]-[1,1'-biphenyl]-3-carboxylic acid;
• 4-bromo-2-[(6-methylpyridin-2-yl)carbamoyl]benzoic acid;
• 4-chloro-2-[(6-methylpyridin-2-yl)carbamoyl]benzoic acid;
• 4-methyl-2-[(6-methylpyridin-2-yl)carbamoyl]benzoic acid;
• 2-[(6-methylpyridin-2-yl)carbamoyl]-4-(trifluoromethyl)benzoic acid;
• 4,5-dichloro-2-[(6-methylpyridin-2-yl)carbamoyl]benzoic acid;
• 4,5-dimethyl-2-[(6-methylpyridin-2-yl)carbamoyl]benzoic acid;
• 3-methyl-2-[(6-methylpyridin-2-yl)carbamoyl]benzoic acid;
• 5-bromo-2-[(butan-2-yl)carbamoyl]benzoic acid;
• 5-bromo-2-[(propan-2-yl)carbamoyl]benzoic acid;
• 5-bromo-2-(phenylcarbamoyl)benzoic acid;
• 5-bromo-2-[(3-methylphenyl)carbamoyl]benzoic acid;
• 5-bromo-2-[(pyridin-2-yl)carbamoyl]benzoic acid;
• 5-bromo-2-[(6-chloropyridin-2-yl)carbamoyl]benzoic acid;
• 5-bromo-2-[(4-methylpyridin-2-yl)carbamoyl]benzoic acid;
• 5-bromo-2-[(3-methylpyridin-2-yl)carbamoyl]benzoic acid;
• 5-bromo-2-[(6-methylpyridin-3-yl)carbamoyl]benzoic acid;
• 5-bromo-2-[(2-methylpyridin-4-yl)carbamoyl]benzoic acid;
• 5-bromo-2-[(2-methylpyrimidin-4-yl)carbamoyl]benzoic acid;
• 2-[(6-methoxypyridin-2-yl)carbamoyl]-5-(trifluoromethyl)benzoic acid;
• 2-[(5,6-dimethylpyridin-2-yl)carbamoyl]-5-(trifluoromethyl)benzoic acid;
• 2-[(5,6,7,8-tetrahydroquinolin-2-yl)carbamoyl]-5-(trifluoromethyl)benzoic acid;
• 1-benzyl-3-tert-butyl-1H-pyrazole-5-carboxylic acid;
• 3-tert-butyl-1-[(3,5-dichlorophenyl)methyl]-1H-pyrazole-5-carboxylic acid;
• 3-tert-butyl-1-{[6-(1H-pyrazol-1-yl)pyridin-3-yl]methyl}-1H-pyrazole-5-carboxylic acid;
• 3-tert-butyl-1-[(6-phenoxypyridin-3-yl)methyl]-1H-pyrazole-5-carboxylic acid;
• 3-tert-butyl-1-methyl-1H-pyrazole-5-carboxylic acid;
• 3-tert-butyl-1-phenyl-1H-pyrazole-5-carboxylic acid;
• 3-tert-butyl-1-(2-phenylethyl)-1H-pyrazole-5-carboxylic acid;
• 3-tert-butyl-1-[(5-phenyl-1,3,4-oxadiazol-2-yl)methyl]-1H-pyrazole-5-carboxylic acid;
• 1-benzyl-3-(2,2-dimethylpropyl)-1H-pyrazole-5-carboxylic acid;
• 1-(2-phenylethyl)-3-(propan-2-yl)-1H-pyrazole-5-carboxylic acid;
• 1-benzyl-3-(3,3-dimethylbutyl)-1H-pyrazole-5-carboxylic acid;
• (2S)-2-[(3,5-dichlorophenyl)formamido]-5,5-dimethylhexanoic acid;
• (2S)-2-[(4-chloro-1H-pyrrol-2-yl)formamido]-5,5-dimethylhexanoic acid;
• (2S)-5,5-dimethyl-2-[(6-phenoxypyridin-3-yl)formamido]hexanoic acid;
• (2S)-2-[(3,4-dichlorophenyl)formamido]-4-methylpentanoic acid;
• (2S)-2-[(3,5-dichlorophenyl)formamido]-4-methylpentanoic acid;
• (2S)-2-[(3-chlorophenyl)formamido]-4-methylpentanoic acid;
• (2S)-2-[(3-methoxyphenyl)formamido]-4-methylpentanoic acid;
• (2S)-2-{2-[(4-chlorophenyl)methyl]-2-methylpropanamido}-4-methylpentanoic acid;
• (2S)-4-methyl-2-[(1-methyl-1H-pyrazol-4-yl)formamido]pentanoic acid;
• (2S)-4-methyl-2-[(quinoxalin-2-yl)formamido]pentanoic acid;
• (2S)-2-(cyclopentylformamido)-4-methylpentanoic acid;
• (2S)-2-({4-[(3,5-dichlorophenyl)methyl]morpholin-2-yl}formamido)-4-methylpentanoic acid;
• (2S)-2-[(3,5-dichlorophenyl)formamido]-4-methylpent-4-enoic acid;
• (2S)-3-cyclopropyl-2-[(3,5-dichlorophenyl)formamido]propanoic acid;
• (2S)-3-cyclohexyl-2-[(3,5-dichlorophenyl)formamido]propanoic acid;
• (2S)-2-[(3,5-dichlorophenyl)formamido]-4,4-dimethylpentanoic acid;
• (2S)-3-{bicyclo[1.1.1]pentan-1-yl}-2-[(3,5-dichlorophenyl)formamido]propanoic acid;
• (2S)-2-[(3,5-dichlorophenyl)formamido]-3-phenylpropanoic acid;
• (2S)-3-(tert-butoxy)-2-[(3,5-dichlorophenyl)formamido]propanoic acid;
• (2S)-3-(tert-butoxy)-2-[(3,5-dichlorophenyl)formamido]butanoic acid;
• 3,5-dichloro-N-[3,3-dimethyl-1-(2H-1,2,3,4-tetrazol-5-yl)butyl]benzamide;
• (2S)-2-{[1-(3,5-dichlorophenyl)-2,2,2-trifluoroethyl]amino}-4-methylpentanoic acid;
• (2S)-4,4-dimethyl-2-[(6-phenoxypyridin-3-yl)formamido]pentanoic acid,
or a pharmaceutically acceptable salt, solvate, hydrate, tautomer, optical isomer, N-oxide, and/or prodrug thereof, preferably the sortilin antagonist is
• 5-bromo-2-[(6-methylpyridin-2-yl)carbamoyl]benzoic acid;
• 5-chloro-2-[(6-methylpyridin-2-yl)carbamoyl]benzoic acid;
• 5-methyl-2-[(6-methylpyridin-2-yl)carbamoyl]benzoic acid;
• 2-[(6-methylpyridin-2-yl)carbamoyl]-5-(propan-2-yl)benzoic acid;
• 2-[(6-methylpyridin-2-yl)carbamoyl]-5-(trifluoromethyl)benzoic acid;
• 2-[(6-methylpyridin-2-yl)carbamoyl]-5-nitrobenzoic acid;
• 4-bromo-2-[(6-methylpyridin-2-yl)carbamoyl]benzoic acid;
• 2-[(6-methylpyridin-2-yl)carbamoyl]-4-(trifluoromethyl)benzoic acid;
• 4,5-dichloro-2-[(6-methylpyridin-2-yl)carbamoyl]benzoic acid;
• 4,5-dimethyl-2-[(6-methylpyridin-2-yl)carbamoyl]benzoic acid;
• 5-bromo-2-[(3-methylphenyl)carbamoyl]benzoic acid;
• 5-bromo-2-[(pyridin-2-yl)carbamoyl]benzoic acid;
• 5-bromo-2-[(6-chloropyridin-2-yl)carbamoyl]benzoic acid;
• 5-bromo-2-[(4-methylpyridin-2-yl)carbamoyl]benzoic acid;
• 5-bromo-2-[(2-methylpyridin-4-yl)carbamoyl]benzoic acid;
• 5-bromo-2-[(2-methylpyrimidin-4-yl)carbamoyl]benzoic acid;
• 2-[(6-methoxypyridin-2-yl)carbamoyl]-5-(trifluoromethyl)benzoic acid;
• 2-[(5,6-dimethylpyridin-2-yl)carbamoyl]-5-(trifluoromethyl)benzoic acid;
• 2-[(5,6,7,8-tetrahydroquinolin-2-yl)carbamoyl]-5-(trifluoromethyl)benzoic acid;
• 1-benzyl-3-tert-butyl-1H-pyrazole-5-carboxylic acid;
• 3-tert-butyl-1-[(3,5-dichlorophenyl)methyl]-1H-pyrazole-5-carboxylic acid;
• 3-tert-butyl-1-[(6-phenoxypyridin-3-yl)methyl]-1H-pyrazole-5-carboxylic acid;
• 3-tert-butyl-1-[(5-phenyl-1,3,4-oxadiazol-2-yl)methyl]-1H-pyrazole-5-carboxylic acid;
• (2S)-2-[(3,5-dichlorophenyl)formamido]-5,5-dimethylhexanoic acid;
• (2S)-2-[(4-chloro-1H-pyrrol-2-yl)formamido]-5,5-dimethylhexanoic acid;
• (2S)-5,5-dimethyl-2-[(6-phenoxypyridin-3-yl)formamido]hexanoic acid;
• (2S)-2-[(3,5-dichlorophenyl)formamido]-4-methylpentanoic acid;
• (2S)-2-({4-[(3,5-dichlorophenyl)methyl]morpholin-2-yl}formamido)-4-methylpentanoic acid;
• (2S)-3-cyclohexyl-2-[(3,5-dichlorophenyl)formamido]propanoic acid;
• (2S)-2-[(3,5-dichlorophenyl)formamido]-4,4-dimethylpentanoic acid;
• (2S)-3-(tert-butoxy)-2-[(3,5-dichlorophenyl)formamido]propanoic acid;
• (2S)-4,4-dimethyl-2-[(6-phenoxypyridin-3-yl)formamido]pentanoic acid;
or a pharmaceutically acceptable salt, solvate, hydrate, tautomer, optical isomer, N-oxide, and/or prodrug thereof.

14. The sortilin antagonist for use according to any one of claims 1 to 4, wherein the sortilin antagonist is an anti-sortilin antibody or an antigen-binding fragment thereof, preferably wherein the anti-sortilin antibody or the antigen-binding fragment thereof binds to at least one amino acid of any of the amino acid sequences according to SEQ ID NO: 1 to 14, or at least one amino acid of the amino acid sequence TGL; preferably wherein the anti-sortilin antibody or the antigen-binding fragment thereof is a polyclonal antibody or a monoclonal antibody; and/or wherein the anti-sortilin antibody or the antigen-binding fragment thereof is a human anti-sortilin antibody, a goat anti-sortilin antibody, a rabbit anti-sortilin antibody or an IgG antibody.

15. A pharmaceutical formulation comprising a sortilin antagonist as defined in any of claims 1 to 14 and one or more pharmaceutically acceptable carriers or excipients, for use in the treatment or prevention of hearing loss, preferably wherein the hearing loss is noise-induced hearing loss, ototoxicity-induced hearing loss, age-induced hearing loss, idiopathic hearing loss, tinnitus or sudden hearing loss; preferably wherein the pharmaceutical formulation is a gel formulation, even more preferably wherein the gel formulation is a Poloxamer gel formulation.


SEQUENCE LISTING

<110>  Insusense ApS

<120>  COMPOUNDS

<130>  RMC00192EP

<140>
<141>

<160>  14

<170>  PatentIn version 3.5

<210>  1
<211>  831
<212>  PRT
<213>  Homo sapiens

<400>  1

<210> 2
<211> 830
<212> PRT
<213> Homo sapiens

<400> 2

Met Glu Arg Pro Trp Gly Ala Ala Asp Gly Leu Ser Arg Trp Pro His
1               5                   10                  15

Gly Leu Gly Leu Leu Leu Leu Leu Gln Leu Leu Pro Pro Ser Thr Leu
            20                  25                  30

Ser Gln Asp Arg Leu Asp Ala Pro Pro Pro Pro Ala Ala Pro Leu Pro
        35                  40                  45

Arg Trp Ser Gly Pro Ile Gly Val Ser Trp Gly Leu Arg Ala Ala Ala
    50                  55                  60

Ala Gly Gly Ala Phe Pro Arg Gly Gly Arg Trp Arg Arg Ser Ala Pro
65                  70                  75                  80

Gly Glu Asp Glu Glu Cys Gly Arg Val Arg Asp Phe Val Ala Lys Leu
                85                  90                  95

Ala Asn Asn Thr His Gln His Val Phe Asp Asp Leu Arg Gly Ser Val
            100                 105                 110

Ser Leu Ser Trp Val Gly Asp Ser Thr Gly Val Ile Leu Val Leu Thr
        115                 120                 125

Thr Phe His Val Pro Leu Val Ile Met Thr Phe Gly Gln Ser Lys Leu
    130                 135                 140

Tyr Arg Ser Glu Asp Tyr Gly Lys Asn Phe Lys Asp Ile Thr Asp Leu
145                 150                 155                 160

Ile Asn Asn Thr Phe Ile Arg Thr Glu Phe Gly Met Ala Ile Gly Pro
                165                 170                 175

Glu Asn Ser Gly Lys Val Val Leu Thr Ala Glu Val Ser Gly Gly Ser
            180                 185                 190

Arg Gly Gly Arg Ile Phe Arg Ser Ser Asp Phe Ala Lys Asn Phe Val
        195                 200                 205

Gln Thr Asp Leu Pro Phe His Pro Leu Thr Gln Met Met Tyr Ser Pro
    210                 215                 220

Gln Asn Ser Asp Tyr Leu Leu Ala Leu Ser Thr Glu Asn Gly Leu Trp
225                 230                 235                 240

Val Ser Lys Asn Phe Gly Gly Lys Trp Glu Glu Ile His Lys Ala Val
                245                 250                 255

Cys Leu Ala Lys Trp Gly Ser Asp Asn Thr Ile Phe Phe Thr Thr Tyr
            260                 265                 270

Ala Asn Gly Ser Cys Thr Asp Leu Gly Ala Leu Glu Leu Trp Arg Thr
        275                 280                 285

Ser Asp Leu Gly Lys Ser Phe Lys Thr Ile Gly Val Lys Ile Tyr Ser
    290                 295                 300

Phe Gly Leu Gly Gly Arg Phe Leu Phe Ala Ser Val Met Ala Asp Lys
305                 310                 315                 320

Asp Thr Thr Arg Arg Ile His Val Ser Thr Asp Gln Gly Asp Thr Trp
                325                 330                 335

Ser Met Ala Gln Leu Pro Ser Val Gly Gln Glu Gln Phe Tyr Ser Ile
            340                 345                 350

Leu Ala Ala Asn Asp Asp Met Val Phe Met His Val Asp Glu Pro Gly
        355                 360                 365

Asp Thr Gly Phe Gly Thr Ile Phe Thr Ser Asp Asp Arg Gly Ile Val
    370                 375                 380

Tyr Ser Lys Ser Leu Asp Arg His Leu Tyr Thr Thr Thr Gly Gly Glu
385                 390                 395                 400

Thr Asp Phe Thr Asn Val Thr Ser Leu Arg Gly Val Tyr Ile Thr Ser
                405                 410                 415

Val Leu Ser Glu Asp Asn Ser Ile Gln Thr Met Ile Thr Phe Asp Gln
            420                 425                 430

Gly Gly Arg Trp Thr His Leu Arg Lys Pro Glu Asn Ser Glu Cys Asp
        435                 440                 445

Ala Thr Ala Lys Asn Lys Asn Glu Cys Ser Leu His Ile His Ala Ser
    450                 455                 460

Tyr Ser Ile Ser Gln Lys Leu Asn Val Pro Met Ala Pro Leu Ser Glu
465                 470                 475                 480

Pro Asn Ala Val Gly Ile Val Ile Ala His Gly Ser Val Gly Asp Ala
                485                 490                 495

Ile Ser Val Met Val Pro Asp Val Tyr Ile Ser Asp Asp Gly Gly Tyr
            500                 505                 510

Ser Trp Thr Lys Met Leu Glu Gly Pro His Tyr Tyr Thr Ile Leu Asp
        515                 520                 525

Ser Gly Gly Ile Ile Val Ala Ile Glu His Ser Ser Arg Pro Ile Asn
    530                 535                 540

Val Ile Lys Phe Ser Thr Asp Glu Gly Gln Cys Trp Gln Thr Tyr Thr
545                 550                 555                 560

Phe Thr Arg Asp Pro Ile Tyr Phe Thr Gly Leu Ala Ser Glu Pro Gly

                565                 570                 575

Ala Arg Ser Met Asn Ile Ser Ile Trp Gly Phe Thr Glu Ser Phe Leu
            580                 585                 590

Thr Ser Gln Trp Val Ser Tyr Thr Ile Asp Phe Lys Asp Ile Leu Glu
        595                 600                 605

Arg Asn Cys Glu Glu Lys Asp Tyr Thr Ile Trp Leu Ala His Ser Thr
    610                 615                 620

Asp Pro Glu Asp Tyr Glu Asp Gly Cys Ile Leu Gly Tyr Lys Glu Gln
625                 630                 635                 640

Phe Leu Arg Leu Arg Lys Ser Ser Val Cys Gln Asn Gly Arg Asp Tyr
                645                 650                 655

Val Val Thr Lys Gln Pro Ser Ile Cys Leu Cys Ser Leu Glu Asp Phe
            660                 665                 670

Leu Cys Asp Phe Gly Tyr Tyr Arg Pro Glu Asn Asp Ser Lys Cys Val
        675                 680                 685

Glu Gln Pro Glu Leu Lys Gly His Asp Leu Glu Phe Cys Leu Tyr Gly
    690                 695                 700

Arg Glu Glu His Leu Thr Thr Asn Gly Tyr Arg Lys Ile Pro Gly Asp
705                 710                 715                 720

Lys Cys Gln Gly Gly Val Asn Pro Val Arg Glu Val Lys Asp Leu Lys
                725                 730                 735

Lys Lys Cys Thr Ser Asn Phe Leu Ser Pro Glu Lys Gln Asn Ser Lys
            740                 745                 750

Ser Asn Ser Val Pro Ile Ile Leu Ala Ile Val Gly Leu Met Leu Val
        755                 760                 765

Thr Val Val Ala Gly Val Leu Ile Val Lys Lys Tyr Val Cys Gly Gly
    770                 775                 780

Arg Phe Leu Val His Arg Tyr Ser Val Leu Gln Gln His Ala Glu Ala
785                 790                 795                 800

Asn Gly Val Asp Gly Val Asp Ala Leu Asp Thr Ala Ser His Thr Asn
                805                 810                 815

Lys Ser Gly Tyr His Asp Asp Ser Asp Glu Asp Leu Leu Glu
            820                 825                 830

<210>  3
<211>  694
<212>  PRT
<213>  Homo sapiens

<400>  3

Met Thr Phe Gly Gln Ser Lys Leu Tyr Arg Ser Glu Asp Tyr Gly Lys
1               5                   10                  15

Asn Phe Lys Asp Ile Thr Asp Leu Ile Asn Asn Thr Phe Ile Arg Thr
            20                  25                  30

Glu Phe Gly Met Ala Ile Gly Pro Glu Asn Ser Gly Lys Val Val Leu
        35                  40                  45

Thr Ala Glu Val Ser Gly Gly Ser Arg Gly Gly Arg Ile Phe Arg Ser
    50                  55                  60

Ser Asp Phe Ala Lys Asn Phe Val Gln Thr Asp Leu Pro Phe His Pro
65                  70                  75                  80

Leu Thr Gln Met Met Tyr Ser Pro Gln Asn Ser Asp Tyr Leu Leu Ala
                85                  90                  95

Leu Ser Thr Glu Asn Gly Leu Trp Val Ser Lys Asn Phe Gly Gly Lys
            100                 105                 110

Trp Glu Glu Ile His Lys Ala Val Cys Leu Ala Lys Trp Gly Ser Asp
        115                 120                 125

Asn Thr Ile Phe Phe Thr Thr Tyr Ala Asn Gly Ser Cys Thr Asp Leu
    130                 135                 140

Gly Ala Leu Glu Leu Trp Arg Thr Ser Asp Leu Gly Lys Ser Phe Lys
145                 150                 155                 160

Thr Ile Gly Val Lys Ile Tyr Ser Phe Gly Leu Gly Gly Arg Phe Leu
                165                 170                 175

Phe Ala Ser Val Met Ala Asp Lys Asp Thr Thr Arg Arg Ile His Val
            180                 185                 190

Ser Thr Asp Gln Gly Asp Thr Trp Ser Met Ala Gln Leu Pro Ser Val
        195                 200                 205

Gly Gln Glu Gln Phe Tyr Ser Ile Leu Ala Ala Asn Asp Asp Met Val
    210                 215                 220

Phe Met His Val Asp Glu Pro Gly Asp Thr Gly Phe Gly Thr Ile Phe
225                 230                 235                 240

Thr Ser Asp Asp Arg Gly Ile Val Tyr Ser Lys Ser Leu Asp Arg His
                245                 250                 255

Leu Tyr Thr Thr Thr Gly Gly Glu Thr Asp Phe Thr Asn Val Thr Ser
            260                 265                 270

Leu Arg Gly Val Tyr Ile Thr Ser Val Leu Ser Glu Asp Asn Ser Ile
        275                 280                 285

Gln Thr Met Ile Thr Phe Asp Gln Gly Gly Arg Trp Thr His Leu Arg
    290                 295                 300

Lys Pro Glu Asn Ser Glu Cys Asp Ala Thr Ala Lys Asn Lys Asn Glu
305                 310                 315                 320

Cys Ser Leu His Ile His Ala Ser Tyr Ser Ile Ser Gln Lys Leu Asn
                325                 330                 335

Val Pro Met Ala Pro Leu Ser Glu Pro Asn Ala Val Gly Ile Val Ile
            340                 345                 350

Ala His Gly Ser Val Gly Asp Ala Ile Ser Val Met Val Pro Asp Val
        355                 360                 365

Tyr Ile Ser Asp Asp Gly Gly Tyr Ser Trp Thr Lys Met Leu Glu Gly
    370                 375                 380

Pro His Tyr Tyr Thr Ile Leu Asp Ser Gly Gly Ile Ile Val Ala Ile
385                 390                 395                 400

Glu His Ser Ser Arg Pro Ile Asn Val Ile Lys Phe Ser Thr Asp Glu
                405                 410                 415

Gly Gln Cys Trp Gln Thr Tyr Thr Phe Thr Arg Asp Pro Ile Tyr Phe
            420                 425                 430

Thr Gly Leu Ala Ser Glu Pro Gly Ala Arg Ser Met Asn Ile Ser Ile
        435                 440                 445

Trp Gly Phe Thr Glu Ser Phe Leu Thr Ser Gln Trp Val Ser Tyr Thr
    450                 455                 460

Ile Asp Phe Lys Asp Ile Leu Glu Arg Asn Cys Glu Glu Lys Asp Tyr
465                 470                 475                 480

Thr Ile Trp Leu Ala His Ser Thr Asp Pro Glu Asp Tyr Glu Asp Gly
                485                 490                 495

Cys Ile Leu Gly Tyr Lys Glu Gln Phe Leu Arg Leu Arg Lys Ser Ser
            500                 505                 510

Val Cys Gln Asn Gly Arg Asp Tyr Val Val Thr Lys Gln Pro Ser Ile
        515                 520                 525

Cys Leu Cys Ser Leu Glu Asp Phe Leu Cys Asp Phe Gly Tyr Tyr Arg
    530                 535                 540

Pro Glu Asn Asp Ser Lys Cys Val Glu Gln Pro Glu Leu Lys Gly His
545                 550                 555                 560

Asp Leu Glu Phe Cys Leu Tyr Gly Arg Glu Glu His Leu Thr Thr Asn
                565                 570                 575

Gly Tyr Arg Lys Ile Pro Gly Asp Lys Cys Gln Gly Gly Val Asn Pro
            580                 585                 590

Val Arg Glu Val Lys Asp Leu Lys Lys Lys Cys Thr Ser Asn Phe Leu
        595                 600                 605

Ser Pro Glu Lys Gln Asn Ser Lys Ser Asn Ser Val Pro Ile Ile Leu
    610                 615                 620

Ala Ile Val Gly Leu Met Leu Val Thr Val Val Ala Gly Val Leu Ile
625                 630                 635                 640

Val Lys Lys Tyr Val Cys Gly Gly Arg Phe Leu Val His Arg Tyr Ser
                645                 650                 655

Val Leu Gln Gln His Ala Glu Ala Asn Gly Val Asp Gly Val Asp Ala
            660                 665                 670

Leu Asp Thr Ala Ser His Thr Asn Lys Ser Gly Tyr His Asp Asp Ser
        675                 680                 685

Asp Glu Asp Leu Leu Glu
    690

<210>  4
<211>  723

<212>  PRT
<213>  Homo sapiens

<400>  4

Gln Asp Arg Leu Asp Ala Pro Pro Pro Pro Ala Ala Pro Leu Pro Arg
1               5                   10                  15

Trp Ser Gly Pro Ile Gly Val Ser Trp Gly Leu Arg Ala Ala Ala Ala
            20                  25                  30

Gly Gly Ala Phe Pro Arg Gly Gly Arg Trp Arg Arg Ser Ala Pro Gly
        35                  40                  45

Glu Asp Glu Glu Cys Gly Arg Val Arg Asp Phe Val Ala Lys Leu Ala
    50                  55                  60

Asn Asn Thr His Gln His Val Phe Asp Asp Leu Arg Gly Ser Val Ser
65                  70                  75                  80

Leu Ser Trp Val Gly Asp Ser Thr Gly Val Ile Leu Val Leu Thr Thr
                85                  90                  95

Phe His Val Pro Leu Val Ile Met Thr Phe Gly Gln Ser Lys Leu Tyr
            100                 105                 110

Arg Ser Glu Asp Tyr Gly Lys Asn Phe Lys Asp Ile Thr Asp Leu Ile
        115                 120                 125

Asn Asn Thr Phe Ile Arg Thr Glu Phe Gly Met Ala Ile Gly Pro Glu
    130                 135                 140

Asn Ser Gly Lys Val Val Leu Thr Ala Glu Val Ser Gly Gly Ser Arg
145                 150                 155                 160

Gly Gly Arg Ile Phe Arg Ser Ser Asp Phe Ala Lys Asn Phe Val Gln
                165                 170                 175

Thr Asp Leu Pro Phe His Pro Leu Thr Gln Met Met Tyr Ser Pro Gln
            180                 185                 190

Asn Ser Asp Tyr Leu Leu Ala Leu Ser Thr Glu Asn Gly Leu Trp Val
        195                 200                 205

Ser Lys Asn Phe Gly Gly Lys Trp Glu Glu Ile His Lys Ala Val Cys
    210                 215                 220

Leu Ala Lys Trp Gly Ser Asp Asn Thr Ile Phe Phe Thr Thr Tyr Ala
225                 230                 235                 240

Asn Gly Ser Cys Lys Ala Asp Leu Gly Ala Leu Glu Leu Trp Arg Thr
                245                 250                 255

Ser Asp Leu Gly Lys Ser Phe Lys Thr Ile Gly Val Lys Ile Tyr Ser
            260                 265                 270

Phe Gly Leu Gly Gly Arg Phe Leu Phe Ala Ser Val Met Ala Asp Lys
        275                 280                 285

Asp Thr Thr Arg Arg Ile His Val Ser Thr Asp Gln Gly Asp Thr Trp
    290                 295                 300

Ser Met Ala Gln Leu Pro Ser Val Gly Gln Glu Gln Phe Tyr Ser Ile
305                 310                 315                 320

Leu Ala Ala Asn Asp Asp Met Val Phe Met His Val Asp Glu Pro Gly
                325                 330                 335

Asp Thr Gly Phe Gly Thr Ile Phe Thr Ser Asp Asp Arg Gly Ile Val
            340                 345                 350

Tyr Ser Lys Ser Leu Asp Arg His Leu Tyr Thr Thr Thr Gly Gly Glu
        355                 360                 365

Thr Asp Phe Thr Asn Val Thr Ser Leu Arg Gly Val Tyr Ile Thr Ser
    370                 375                 380

Val Leu Ser Glu Asp Asn Ser Ile Gln Thr Met Ile Thr Phe Asp Gln
385                 390                 395                 400

Gly Gly Arg Trp Thr His Leu Arg Lys Pro Glu Asn Ser Glu Cys Asp
                405                 410                 415

Ala Thr Ala Lys Asn Lys Asn Glu Cys Ser Leu His Ile His Ala Ser
            420                 425                 430

Tyr Ser Ile Ser Gln Lys Leu Asn Val Pro Met Ala Pro Leu Ser Glu
        435                 440                 445

Pro Asn Ala Val Gly Ile Val Ile Ala His Gly Ser Val Gly Asp Ala
    450                 455                 460

Ile Ser Val Met Val Pro Asp Val Tyr Ile Ser Asp Asp Gly Gly Tyr
465                 470                 475                 480

Ser Trp Thr Lys Met Leu Glu Gly Pro His Tyr Tyr Thr Ile Leu Asp

                485                 490                 495

Ser Gly Gly Ile Ile Val Ala Ile Glu His Ser Ser Arg Pro Ile Asn
            500                 505                 510

Val Ile Lys Phe Ser Thr Asp Glu Gly Gln Cys Trp Gln Thr Tyr Thr
        515                 520                 525

Phe Thr Arg Asp Pro Ile Tyr Phe Thr Gly Leu Ala Ser Glu Pro Gly
    530                 535                 540

Ala Arg Ser Met Asn Ile Ser Ile Trp Gly Phe Thr Glu Ser Phe Leu
545                 550                 555                 560

Thr Ser Gln Trp Val Ser Tyr Thr Ile Asp Phe Lys Asp Ile Leu Glu
                565                 570                 575

Arg Asn Cys Glu Glu Lys Asp Tyr Thr Ile Trp Leu Ala His Ser Thr
            580                 585                 590

Asp Pro Glu Asp Tyr Glu Asp Gly Cys Ile Leu Gly Tyr Lys Glu Gln
        595                 600                 605

Phe Leu Arg Leu Arg Lys Ser Ser Val Cys Gln Asn Gly Arg Asp Tyr
    610                 615                 620

Val Val Thr Lys Gln Pro Ser Ile Cys Leu Cys Ser Leu Glu Asp Phe
625                 630                 635                 640

Leu Cys Asp Phe Gly Tyr Tyr Arg Pro Glu Asn Asp Ser Lys Cys Val
                645                 650                 655

Glu Gln Pro Glu Leu Lys Gly His Asp Leu Glu Phe Cys Leu Tyr Gly
            660                 665                 670

Arg Glu Glu His Leu Thr Thr Asn Gly Tyr Arg Lys Ile Pro Gly Asp
        675                 680                 685

Lys Cys Gln Gly Gly Val Asn Pro Val Arg Glu Val Lys Asp Leu Lys
    690                 695                 700

Lys Lys Cys Thr Ser Asn Phe Leu Ser Pro Glu Lys Gln Asn Ser Lys
705                 710                 715                 720

Ser Asn Ser

<210>  5
<211>  36
<212>  PRT
<213>  Homo sapiens

<400>  5

Ser Val Met Ala Asp Lys Asp Thr Thr Arg Arg Ile His Val Ser Thr
1               5                   10                  15

Asp Gln Gly Asp Thr Trp Ser Met Ala Gln Leu Pro Ser Val Gly Gln
            20                  25                  30

Glu Gln Phe Tyr
        35

<210>  6
<211>  5
<212>  PRT
<213>  Homo sapiens

<400>  6

Gly Ser Val Ser Leu
1               5

<210>  7
<211>  29
<212>  PRT
<213>  Homo sapiens

<400>  7

Ser Ile Ser Gln Lys Leu Asn Val Pro Met Ala Pro Leu Ser Glu Pro
1               5                   10                  15

Asn Ala Val Gly Ile Val Ile Ala His Gly Ser Val Gly
            20                  25

<210>  8
<211>  12
<212>  PRT
<213>  Homo sapiens

<400>  8

Arg Ile Phe Arg Ser Ser Asp Phe Ala Lys Asn Phe
1               5                   10

<210>  9
<211>  36
<212>  PRT
<213>  Homo sapiens

<400>  9

Ser Val Met Ala Asp Lys Asp Thr Thr Arg Arg Ile His Val Ser Thr
1               5                   10                  15

Asp Gln Gly Asp Thr Trp Ser Met Ala Gln Leu Pro Ser Val Gly Gln
            20                  25                  30

Glu Gln Phe Tyr
        35

<210>  10
<211>  9
<212>  PRT
<213>  Homo sapiens

<400>  10

Ser Glu Asp Asn Ser Ile Gln Thr Met
1               5

<210>  11
<211>  5
<212>  PRT
<213>  Homo sapiens

<400>  11

Ile His Ala Ser Ile
1               5

<210>  12
<211>  12
<212>  PRT
<213>  Homo sapiens

<400>  12

Val Ile Ala His Gly Ser Val Gly Asp Ala Ile Ser
1               5                   10

<210>  13
<211>  17
<212>  PRT
<213>  Homo sapiens

<400>  13

Gly Tyr Arg Lys Ile Pro Gly Asp Lys Cys Gln Gly Gly Val Asn Pro
1               5                   10                  15

Val

<210>  14
<211>  145
<212>  PRT

<213>  Homo sapiens

<400>  14

Cys Glu Glu Lys Asp Tyr Thr Ile Trp Leu Ala His Ser Thr Asp Pro
1               5                   10                  15

Glu Asp Tyr Glu Asp Gly Cys Ile Leu Gly Tyr Lys Glu Gln Phe Leu
            20                  25                  30

Arg Leu Arg Lys Ser Ser Val Cys Gln Asn Gly Arg Asp Tyr Val Val
        35                  40                  45

Thr Lys Gln Pro Ser Ile Cys Leu Cys Ser Leu Glu Asp Phe Leu Cys
    50                  55                  60

Asp Phe Gly Tyr Tyr Arg Pro Glu Asn Asp Ser Lys Cys Val Glu Gln
65                  70                  75                  80

Pro Glu Leu Lys Gly His Asp Leu Glu Phe Cys Leu Tyr Gly Arg Glu
                85                  90                  95

Glu His Leu Thr Thr Asn Gly Tyr Arg Lys Ile Pro Gly Asp Lys Cys
            100                 105                 110

Gln Gly Gly Val Asn Pro Val Arg Glu Val Lys Asp Leu Lys Lys Lys
        115                 120                 125

Cys Thr Ser Asn Phe Leu Ser Pro Glu Lys Gln Asn Ser Lys Ser Asn
    130                 135                 140

Ser
145